(19) Europäisches Patentamt
European Patent Office
Office européen des brevets

(11) Veröffentlichungsnummer: **0 578 002 A1**

(12) **EUROPÄISCHE PATENTANMELDUNG**

(21) Anmeldenummer: **93109464.3**

(22) Anmeldetag: **14.06.93**

(51) Int. Cl.5: **C07D 233/68**, C07D 403/10, A61K 31/415, C07D 233/54

(30) Priorität: **26.06.92 DE 4220983**

(43) Veröffentlichungstag der Anmeldung: **12.01.94 Patentblatt 94/02**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Müller, Ulrich E., Dr.**
**Claudiusweg 5**
**D-5600 Wuppertal(DE)**
Erfinder: **Dressel, Jürgen, PH.D.**
**Claudiusweg 9**
**D-5600 Wuppertal(DE)**
Erfinder: **Fey, Peter, Dr.**
**Am Eickhof 23**
**D-5600 Wuppertal(DE)**
Erfinder: **Hanko, Rudolf, Dr.**
**Schillerstrasse 23**
**D-4000 Düsseldorf(DE)**
Erfinder: **Hübsch, Walter, Dr.**
**Wildsteig 22**
**D-5600 Wuppertal(DE)**
Erfinder: **Krämer, Thomas, Dr.**
**In den Birken 92a**
**D-5600 Wuppertal(DE)**
Erfinder: **Müller-Gliemann, Matthias, Dr.**
**Laibacher Strasse 10**
**D-5650 Solingen-Ohligs(DE)**
Erfinder: **Beuck, Martin, Dr.**
**Trills 7**
**D-4006 Erkrath 2(DE)**
Erfinder: **Kazda, Stanislav, Prof. Dr.**
**Gellertweg 18**
**D-5600 Wuppertal(DE)**
Erfinder: **Knorr, Andreas, Dr.**
**Trillser Graben 10**
**D-4006 Erkrath 2(DE)**
Erfinder: **Stasch, Johannes-Peter, Dr.**
**Schneewittchenweg 37**
**D-5600 Wuppertal(DE)**
Erfinder: **Wohlfeil, Stefan, Dr.**
**Tucherweg 25**
**D-4010 Hilden(DE)**

(54) **Imidazolyl-substituierte Phenylpropion- und Zimtsäurederivate.**

(57) Imidazolyl-substituierte Phenylpropion- und -zimtsäurederivate werden hergestellt, indem man entsprechende Benzylverbindungen mit Imidazolen umsetzt und gegebenenfalls die Substituenten variiert. Die Imidazolyl-substituierten Phenylpropion- und -zimtsäurederivate können als Wirkstoffe in Arzneimitteln, insbesondere bei der Behandlung von arterieller Hypertonie und Atherosklerose verwendet werden.

EP 0 578 002 A1

Rank Xerox (UK) Business Services

Die Erfindung betrifft Imidazolyl-substituierte Phenylpropion- und -zimtsäurederivate, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, $Na^-$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 102, EP 399 731; EP 399 732, EP 324 347 und EP 253 310 heterocyclische Verbindungen mit A II-antagonistischer Wirkung bekannt.

Ferner werden in den Publikationen JP 02 053 779 und JP 62 039 576 Imidazolbenzylderivate beschrieben, die am Phenylring eine substituierte Vinylgruppe besitzen und die eine peptic ulcer inhibitorische Wirkung besitzen.

Die vorliegende Erfindung betrifft Imidazolyl-substituierte Phenylpropion- und -zimtsäurederivate der allgemeinen Formel (I)

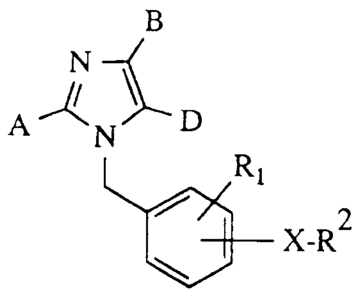

(I),

in welcher

A für geradkettiges oder verzweigtes Alkyl oder Alkenyl mit jeweils bis zu 8 Kohlenstoffatomen steht, oder für Cycloalkyl mit 3 bis 8 Kohlenstoffatomen steht,

B für Wasserstoff, Halogen oder für Perfluoralkyl mit bis zu 5 Kohlenstoffatomen steht,

D für eine Gruppe der Formel $-CH_2-OR^3$ oder $-CO-R^4$ steht,
worin

$R^3$ Wasserstoff oder geradkettiges oder verzweigtes Alkyl mit bis zu 8 Kohlenstoffatomen bedeutet,

$R^4$ Wasserstoff, Hydroxy oder geradkettiges oder verzweigtes Alkoxy mit bis zu 8 Kohlenstoffatomen bedeutet,

X für eine Gruppe der Formel

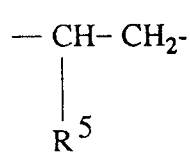 oder 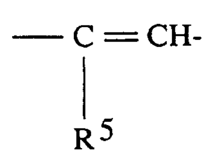

steht,

worin

$R^5$ Cycloalkyl mit 3 bis 8 Kohlenstoffatomen bedeutet, oder Phenyl bedeutet, oder geradkettiges oder verzweigtes Alkyl mit bis zu 8 Kohlenstoffatomen bedeutet, das gegebenenfalls durch Phenyl oder Cycloalkyl mit 3 bis 8 Kohlenstoffatomen substituiert ist.

$R^1$ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

$R^2$ für Carboxy, geradkettiges oder verzweigtes Alkoxycarbonyl mit bis zu 8 Kohlenstoffatomen oder Azido steht, oder für einen Rest der Formel $-NR^6R^7$, $-CO-NR^8R^9$ oder

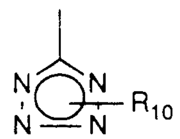

steht.

worin

$R^6$, $R^7$, $R^8$ und $R^9$ gleich oder verschieden sind und Wasserstoff, geradkettiges oder verzweigtes Alkyl mit bis zu 8 Kohlenstoffatomen, Benzyl oder Phenyl bedeuten,

oder

$R^6$ und $R^8$ die oben angegebene Bedeutung haben

und

$R^7$ und/oder $R^9$ eine Gruppe der Formel $-SO_2R^{11}$ oder $-CO-R^{12}$ bedeuten,

worin

$R^{11}$ Trifluormethyl oder geradkettiges oder verzweigtes Alkyl mit bis zu 8 Kohlenstoffatomen bedeutet, das gegebenenfalls durch Phenyl substituiert ist, das seinerseits durch geradkettiges oder verzweigtes Alkyl mit bis zu 6 Kohlenstoffatomen substituiert sein kann, oder Phenyl bedeutet, das gegebenenfalls durch geradkettiges oder verzweigtes Alkyl mit bis zu 6 Kohlenstoffatomen substituiert ist,

$R^{12}$ geradkettiges oder verzweigtes Alkyl mit bis zu 8 Kohlenstoffatomen, Benzyl oder Phenyl bedeutet, das gegebenenfalls durch geradkettiges oder verzweigtes Alkyl mit bis zu 6 Kohlenstoffatomen substituiert ist,

oder

$R^8$ Wasserstoff bedeutet

und

$R^9$ die Gruppe der Formel

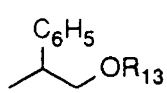

bedeutet,

worin

$R^{13}$ Wasserstoff oder geradkettiges oder verzweigtes Alkyl mit bis zu 6 Kohlenstoffatomen bedeutet,

$R^{10}$ Wasserstoff, geradkettiges oder verzweigtes Alkyl mit bis zu 6 Kohlenstoffatomen oder die Triphenylmethylgruppe bedeutet,

und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Imidazolyl-substituierten Phenylpropion- und -zimtsäurederivate können

Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren als auch Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Hydroxyschutzgruppe im Rahmen der oben angegebenen Definition steht im allgemeinen für eine Schutzgruppe aus der Reihe:
Trimethylsilyl, Triethylsilyl, Triisopropylsilyl, tert.Butyl-dimethylsilyl, tert.-Butyl-diphenylsilyl, Trimethylsilylethoxycarbonyl, Benzyl, Triphenylmethyl(Trityl), Monomethoxytrityl (MMTr), Dimethoxytritryl (DMTr), Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl. Bevorzugt ist Acetyl.

Bevorzugt sind Verbindungen der allgemeinen Formel (I),
in welcher

A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,

B für Wasserstoff, Fluor, Chlor, Brom oder für Perfluoralkyl mit bis zu 4 Kohlenstoffatomen steht,

D für eine Gruppe der Formel $-CH_2OR^3$ oder $-CO-R^4$ steht,
worin

$R^3$ Wasserstoff oder geradkettiges oder verzweigtes Alkyl mit bis zu 6 Kohlenstoffatomen bedeutet,

$R^4$ Wasserstoff, Hydroxy oder geradkettiges oder verzweigtes Alkoxy mit bis zu 6 Kohlenstoffatomen bedeutet,

X für eine Gruppe der Formel

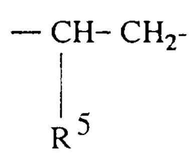 oder 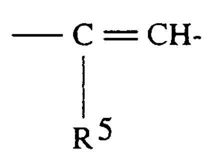

steht,
worin

$R^5$ Cyclopropyl, Cyclopentyl, Cyclohexyl, Cycloheptyl oder Phenyl bedeutet,
oder
geradkettiges oder verzweigtes Alkyl mit bis zu 6 Kohlenstoffatomen bedeutet, das durch Phenyl, Cyclopropyl, Cyclopentyl, Cyclohexyl oder Cycloheptyl substituiert ist,

$R^1$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlen-

stoffatomen steht.

$R^2$ für Carboxy, geradkettiges oder verzweigtes Alkoxycarbonyl mit bis zu 6 Kohlenstoffatomen oder Azido steht, oder
für einen Rest der Formel $-NR^6R^7$, $-CO-NR^8R^9$ oder

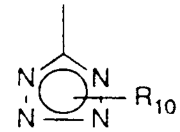

steht,
worin

$R^6$, $R^7$, $R^8$ und $R^9$ gleich oder verschieden sind und Wasserstoff, geradkettiges oder verzweigtes Alkyl mit bis zu 6 Kohlenstoffatomen, Benzyl oder Phenyl bedeuten,
oder

$R^6$ und $R^8$ die oben angegebene Bedeutung haben
und

$R^7$ und/oder $R^9$ eine Gruppe der Formel $-SO_2R^{11}$ oder $-CO-R^{12}$ bedeuten,
worin

$R^{11}$ Trifluormethyl oder geradkettiges oder verzweigtes Alkyl mit bis zu 6 Kohlenstoffatomen bedeutet, das gegebenenfalls durch Phenyl substituiert ist, das seinerseits durch geradkettiges oder verzweigtes Alkyl mit bis zu 4 Kohlenstoffatomen substituiert sein kann, oder
Phenyl bedeutet, das gegebenenfalls durch geradkettiges oder verzweigtes Alkyl mit bis zu 4 Kohlenstoffatomen substituiert ist,

$R^{12}$ geradkettiges oder verzweigtes Alkyl mit bis zu 6 Kohlenstoffatomen, Phenyl, Benzyl oder Tolyl bedeutet,
oder

$R^8$ Wasserstoff bedeutet
und

$R^9$ die Gruppe der Formel

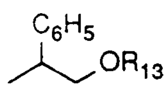

bedeutet,
worin

$R^{13}$ Wasserstoff oder geradkettiges oder verzweigtes Alkyl mit bis zu 4 Kohlenstoffatomen bedeutet.

$R^{10}$ Wasserstoff, geradkettiges oder verzweigtes Alkyl mit bis zu 4 Kohlenstoffatomen oder die Triphenylmethylgruppe bedeutet

und deren Salze.

Besonders bevorzugt sind Verbindungen der allgemeinen Formel (I)
in welcher

A für geradkettiges oder verzweigtes Alkyl oder Alkenyl mit jeweils bis zu 4 Kohlenstoffatomen steht, oder
für Cyclopropyl, Cyclopentyl oder Cyclohexyl steht,

B für Wasserstoff, Fluor, Chlor oder für Perfluoralkyl mit bis zu 2 Kohlenstoffatomen steht,

D für eine Gruppe der Formel $-CH_2OR^3$ oder $-CO-R^4$ steht,
worin

$R^3$ Wasserstoff oder geradkettiges oder verzweigtes Alkyl mit bis zu 4 Kohlenstoffatomen bedeutet,

$R^4$ Wasserstoff, Hydroxy oder geradkettiges oder verzweigtes Alkoxy mit bis zu 4 Kohlenstoffatomen bedeutet,

X für eine Gruppe der Formel

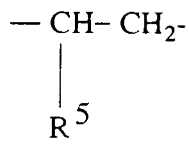 oder 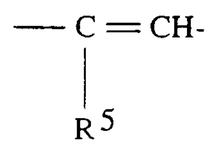

steht,
worin

$R^5$ Cyclopentyl, Cyclohexyl oder Phenyl bedeutet, oder geradkettiges oder verzweigtes Alkyl mit bis zu 4 Kohlenstoffatomen bedeutet, das gegebenenfalls durch Phenyl, Cyclopentyl oder Cyclohexyl substituiert ist,

$R^1$ für Wasserstoff, Fluor, Chlor, Brom, Trifluormethyl, Trifluormethoxy oder Methyl steht,

$R^2$ für Carboxy, geradkettiges oder verzweigtes Alkoxycarbonyl mit bis zu 4 Kohlenstoffatomen oder Azido steht, oder für einen Rest der Formel $-NR^6R^7$, $-CO-NR^8R^9$ oder

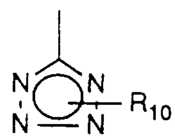

steht,
worin

$R^6$, $R^7$, $R^8$ und $R^9$ gleich oder verschieden sind und Wasserstoff, geradkettiges oder verzweigtes Alkyl mit bis zu 4 Kohlenstoffatomen, Benzyl oder Phenyl bedeuten,
oder

$R^6$ und $R^8$ die oben angegebene Bedeutung haben
und

$R^7$ und/oder $R^9$ eine Gruppe der Formel $-SO_2-R^{11}$ oder $-CO-R^{12}$ bedeuten,
worin

$R^{11}$ Trifluormethyl oder geradkettiges oder verzweigtes Alkyl mit bis zu 4 Kohlenstoffatomen bedeutet, das gegebenenfalls durch Phenyl oder Tolyl substituiert sein kann, oder Phenyl oder Tolyl bedeutet,

$R^{12}$ geradkettiges oder verzweigtes Alkyl mit bis zu 4 Kohlenstoffatomen, Benzyl, Phenyl oder Tolyl bedeutet,
oder

$R^8$ Wasserstoff bedeutet
und

$R^9$ die Gruppe der Formel

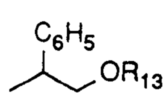

bedeutet,
worin

$R^{13}$ Wasserstoff, Methyl oder Ethyl bedeutet,

$R^{10}$ Wasserstoff, Methyl oder die Triphenylmethylgruppe bedeutet

und deren Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II)

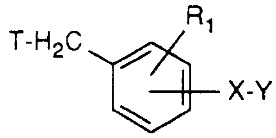

(II),

in welcher

R' und X die oben angegebene Bedeutung haben,

T für eine typische Abgangsgruppe wie beispielsweise Chlor, Brom, Jod, Tosylat oder Mesylat, vorzugsweise für Brom, steht

und

Y für geradkettiges oder verzweigtes ($C_1$-$C_4$)-Alkoxycarbonyl oder für die Triphenylmethyl-tetrazol-1-yl-Gruppe steht,

zunächst mit Imidazolen der allgemeinen Fomel (III)

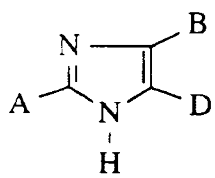

(III),

in welcher

A, B und D die oben angegebene Bedeutung haben,

in inerten Lösemitteln, gegebenenfalls in Anwesenheit einer Base und gegebenenfalls unter Schutzgasatmosphäre zu Verbindungen der allgemeinen Formel (IV)

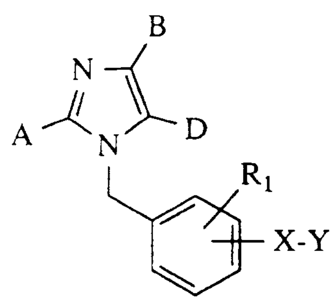

(IV),

in welcher

A, B, D, E, R' und X und Y die oben angegebene Bedeutung haben,

umsetzt,

und im Fall der Säuren ($R^2$ = $CO_2H$) die Ester verseift,

und im Fall der Amine, Amide und Sulfonamide mit Verbindungen der allgemeinen Formel (V)

$H\text{-}NR^{14}R^{15}$ (V),

in welcher

$R^{14}$ und $R^{15}$ den jeweiligen Bedeutungsumfang von $R^6$, $R^7$, $R^8$ und $R^9$ erfassen,

gegebenenfalls in Anwesenheit einer Base und oder eines Hilfsstoffes, beispielsweise eines Dehydratisierungsmittels, in inerten Lösemitteln umsetzt,

und im Fall des freien Tetrazols, die Tritylgruppe mit Säuren, vorzugsweise mit Trifluoressigsäure oder Salzsäure in Dioxan, abspaltet,

und gegebenenfalls die Substituenten A, B, D und R' nach üblichen Methoden, beispielsweise durch Reduktion, Oxidation, Alkylierung oder Hydrolyse einführt oder in andere Gruppen überführt,

und gegebenenfalls die Isomeren trennt, und im Fall der Herstellung der Salze mit einer entsprechenden Base oder Säure umsetzt,

und im Fall der Ester, ausgehend von den aktivierten Carbonsäuren, mit den entsprechenden Alkoholaten umsetzt.

Das erfindungsgemäße Verfahren kann durch folgendes Formelschema beispielhaft erläutert werden:

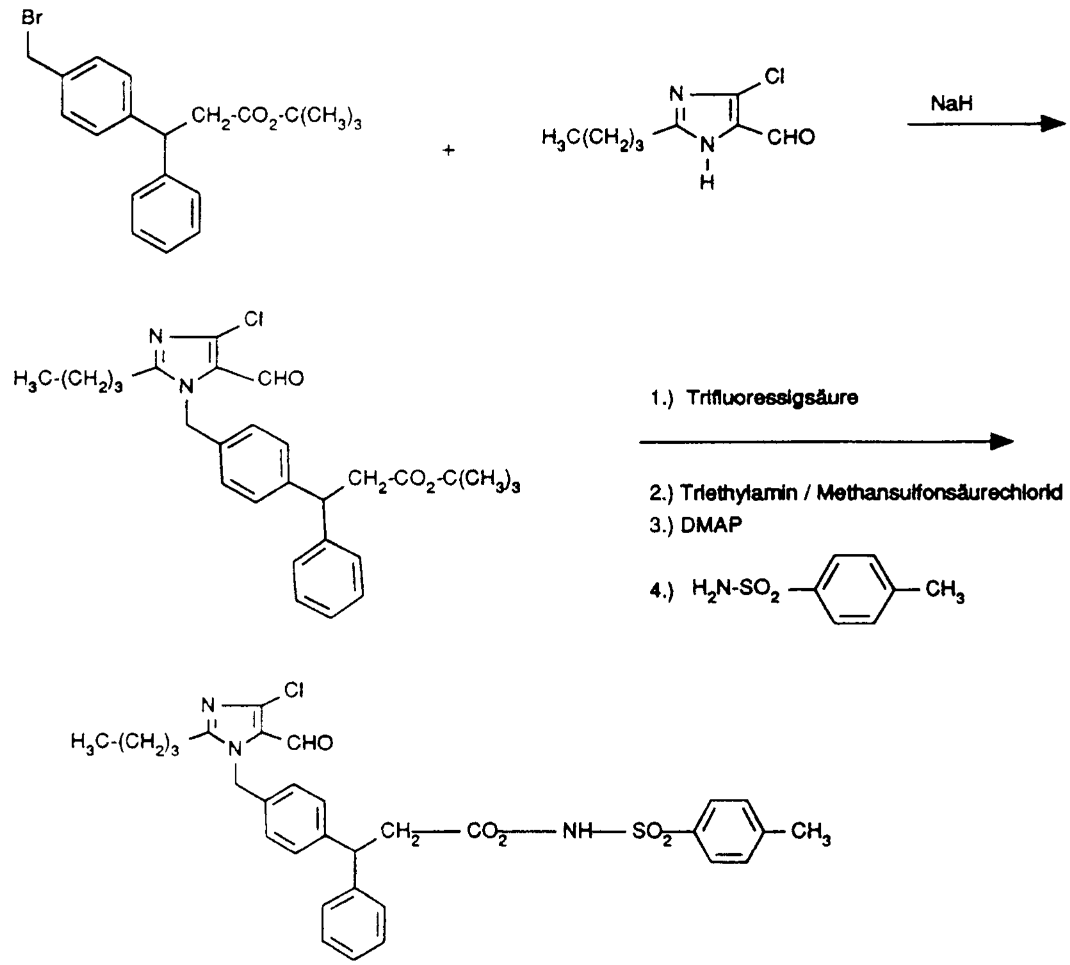


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -30 °C bis +100 °C, bevorzugt von -10 °C bis +60 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid. oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Verseifung Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Hydrolyse mit Säuren wie beispielsweise Trifluoressigsäure, Essigsäure, Chlorwasserstoffsäure, Chlorwasserstoffsäure Dioxan, Bromwasserstoffsäure, Methansulfonsäure, Schwefelsäure oder Perchlorsäure, besonders bevorzugt mit Trifluoressigsäure oder Chlorwasserstoffsäure/Dioxan.

Die Hydrolyse wird im allgemeinen in einem Temperaturbereich von 0°C bis +100°C, bevorzugt von +20°C bis +80°C, durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und die Sulfonamidierung der Verbindungen der allgemeinen Formel (IV) erfolgt im allgemeinen in einem der oben aufgeführten Lösemittel, vorzugsweise in Tetrahydrofüran oder Dichlormethan.

Die Amidierung oder Sulfonamidierung kann ausgehend von den Verbindungen der allgemeinen Formel (IV) gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Die Amidierung oder Sulfonamidierung erfolgt im allgemeinen in einem Temperaturbereich von -50°C bis +80°C, vorzugsweise von -30°C bis +20°C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formel (V), eingesetzt.

Als säurebindende Mittel für die Amidierung oder Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die oben aufgeführte Derivatisierung der Substituenten A, B, D und $R^1$ erfolgt im allgemeinen nach literaturbekannten Methoden, wobei beispielhaft die Reduktion von Aldehyden oder Alkoxycarbonylverbindungen zu Alkoholen (a), die Reduktion von Doppelbindungen (b) und die Alkylierung (c) mit folgendem erläutert werden sollen:

a) Die Reduktion von Alkoxycarbonylverbindungen oder Aldehyden zu den entsprechenden Alkoholen erfolgt im allgemeinen mit Hydriden, wie Lithiumaluminiumhydrid oder Natriumborhydrid, bevorzugt mit Lithiumaluminiumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0 °C bis +150 °C, bevorzugt von +20 °C bis +100 °C, bei Normaldruck.

Die Reduktion einer Doppelbindung erfolgt im allgemeinen durch Hydrierung mit Wasserstoff in Anwesenheit eines Katalysators wie beispielsweise Platin oder Platinoxide, Rhodium, Ruthenium, Chlorotris(triphenylphosphin)rhodium, oder Palladium auf Tierkohle, bevorzugt mit Palladium auf Tierkohle in einem Temperaturbereich von 0 °C bis +150 °C, bevorzugt von +25 °C bis +100 °C.

b) Als Lösemittel für die Hydrierung eignen sich protische Lösemittel wie beispielsweise Methanol, Ethanol und/oder aprotische Lösemittel wie beispielsweise Tetrahydrofüran, Toluol, Dimethylformamid, Methylenchlorid, Dioxan oder Essigester.

Die Hydrierung wird bei einem Druck von 1 bis 300 atm, vorzugsweise bei 1 bis 20 atm, durchgeführt.

c) Die Alkylierung erfolgt im allgemeinen in einem der oben aufgeführten Lösemitteln mit Alkylierungsmitteln wie beispielsweise $(C_1-C_8)$-Alkylhalogenide, Sulfonsäureester oder substituierte oder unsubstituierte $(C_1-C_6)$-Dialkyl- oder $(C_6-C_{10})$-Diarylsulfate, vorzugsweise Methyljodid, p-Toluolsulfonsäureester oder Dimethylsulfat.

Die Verbindungen der allgemeinen Formel (II) sind neu und können hergestellt werden, indem man im Fall, daß der Rest X für die

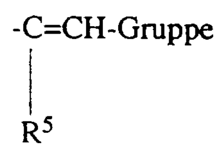

steht,
zunächst Verbindungen der allgemeinen Formel (VI)

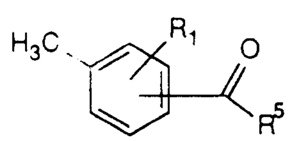

(VI),

in welcher
$R^1$ und $R^5$ die oben angegebene Bedeutung haben,
durch Reduktion nach üblichen Methoden, vorzugsweise mit Natriumhydrid, in einem der oben aufgeführten Lösemitteln, vorzugsweise in Toluol, und durch anschließende Veresterung in die Verbindungen der allgemeinen Formel (VII)

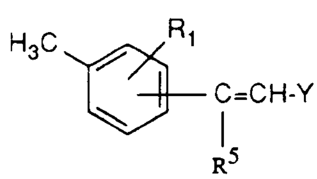

(VII),

in welcher
$R^1$, $R^5$ und Y die oben angegebene Bedeutung haben,

überführt,
und in einem zweiten Schritt eine Bromierung der Methylgruppe, gegebenenfalls in Anwesenheit eines Katalysators durchführt,
und im Fall, daß der Rest X für die

$$\begin{array}{l}-CH-CH_2\text{-Gruppe}\\ \;\;|\\ \;R^5\end{array}$$

steht,
entweder Verbindungen der allgemeinen Formel (VII) nach üblichen Methoden reduziert oder zunächst Verbindungen der allgemeinen Formel (VI) in einer Grignardreaktion, beispielsweise mit ($C_1$-$C_4$)-Alkylmagnesiumhalogeniden, vorzugsweise Bromiden reduziert und gleichzeitig den Rest $R^2$ einführt und anschließend wie oben beschrieben die Bromierung durchführt,
und gegebenenfalls auf den Stufen der allgemeinen Formel (VI) oder (VII) den Substituenten $R^1$ wie oben beschrieben variiert.

Die Reduktion erfolgt entweder durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle, oder Platin, oder aber mit Hydriden in inerten Lösemitteln, gegebenenfalls in Anwesenheit eines Katalysators.

Die Reduktion der Doppelbindung erfolgt in einem Temperaturbereich von 0 °C bis +40 °C, vorzugsweise bei +20 °C und einem Druck von 1 bar.

Die Veresterung erfolgt in einem der oben angegebenen Lösemitteln, vorzugsweise Toluol oder Tetrahydrofuran, nach der oben bereits beschriebenen vorgeschalteten Aktivierung der entsprechenden Carbonsäure, vorzugsweise über die Carbonsäurechloride, und nachträglicher Umsetzung mit dem entsprechenden Alkoholaten, in einem Temperaturbereich von 0 °C bis +60 °C, vorzugsweise bei +10 °C bis +35 °C und Normaldruck.

Die Abspaltung der Magnesiumhalogenid-Gruppe erfolgt nach der für Grignard-Reaktionen üblichen Methode mit wäßriger Ammoniumchloridlösung [vgl. J. March, Advanced Organic Chemistry, Second Edition S. 836].

Die Bromierung wird im allgemeinen in einem Temperaturbereich von +40 °C bis 100 °C, vorzugsweise von +60 °C bis +90 °C und Normaldruck durchgeführt. Sie erfolgt in einem der oben angegebenen Lösemitteln, vorzugsweise mit Tetrachlorkohlenstoff, und mit N-Bromsuccinimid.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0.01 mol bis 0.05 mol, bezogen auf 1 mol der Verbindung der allgemeinen Formel (VI), eingesetzt wird.

Die Verbindungen der allgemeinen Formel (VI) sind größtenteils bekannt oder können nach üblichen Methoden hergestellt werden.

Die Verbindungen der allgemeinen Formel (VIII) sind neu und können beispielsweise wie oben beschrieben hergestellt werden.

Die Verbindungen der allgemeinen Formel (III) sind an sich bekannt oder können nach üblichen Methoden hergestellt werden [vgl. z.B. Beilstein 25, 163; 23, 45; US 4 355 040].

Die Verbindungen der allgemeinen Formel (IV) sind neu und können wie oben beschrieben beispielweise hergestellt werden.

Die Verbindungen der allgemeinen Formel (V) sind bekannt [vgl. z.B. Beilstein 11/104, R.V. Vitzgert, Uspekhi, Khimii 32, 3 (1963); Russian Chem. Rev. 32, 1 (1969); Beilstein 4, 87].

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüber hinaus inhibieren sie die Proliferation von glatten Muskelzellen.

Sie können deshalb in Arzneimitteln zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen,

Funktionsstörungen der Niere und Nebenniere. bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen (Applikationsvolumen pro Einzelgabe = 100 µl): | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration, bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Tabelle A

| Hemmung der Gefäßkontraktion an isolierten Aortenringen von Kaninchen in vitro | |
|---|---|
| $IC_{50}$ (g/ml) gegen Kontraktionen, induziert durch AII | |
| Bsp.Nr.: | $IC_{50}$ [nM] |
| 20 | 360 |
| 30 | 580 |
| 31 | 360 |
| 35 | 650 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3H$-Angiotensin II (3-5 nM), Test-Pufferlösung (50 mM Tris, pH 7,2, 5 mM $MgCl_2$) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Tabelle B

| Bsp.-Nr. | Ki [nM] |
|---|---|
| 30 | 340 |
| 31 | 310 |
| 36 | 150 |
| 37 | 190 |

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Schweinen durch die Media-Explantat-Technik gewonnen werden [R. Ross, J. Cell.

Biol. 50, 172, 1971]. Die Zellen werden in geeigneten Kulturschalen, in der Regel 96-Loch-Platten, ausgesät und für 2 - 3 Tage in Medium 199 mit 7,5 % FCS und 7,5 % NCS, 2 mM L-Glutamin und 15 mmol HEPES, pH 7,4 in 5% $CO_2$ bei 37 °C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

Zur Bestimmung der $IC_{50}$-Werte wird die Wirkstoffkonzentration errechnet, die bei sequentieller Verdünnung des Wirkstoffes halbmaximale Hemmung der durch 1 % FCS hervorgerufenen Thymidininkorporation bewirkt.

Tabelle C

| Bsp.-Nr. | Inhibition [%] bei $10^{-6}$ M |
|---|---|
| 18 | 90 |
| 39 | 59 |
| 40 | 57 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Losemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0.01 bis 0.5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

| Lösemittelgemische | | |
|---|---|---|
| A | = Petrolether : Essigester | = 5:1 |
| B | = Dichlormethan : Methanol | = 50:1 |
| C | = Petrolether : Essigester | = 10:1 |
| D | = Petrolether : Essigester | = 2:1 |
| E | = Petrolether : Essigester | = 1:1 |
| F | = Dichlormethan : Methanol | = 20:1 |
| G | = Dichlormethan : Methanol | = 10:1 |
| H | = Dichlormethan : Methanol | = 5:1 |
| I | = Dichlormethan : Methanol | = 30:1 |
| J | = Petrolether : Ether | = 20:1 |
| K | = Petrolether : Essigester | = 20:1 |

Ausgangsverbindungen

Beispiel I

2-Cyclopentyl-2-(4-methylphenyl)essigsäuremethylester

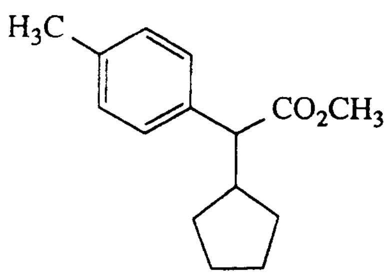

Bei 0°C wird eine Suspension von 5,68 g (189 mmol) Natriumhydrid (80%ig, stabilisiert mit Paraffin) in 200 ml Dimethylformamid unter Feuchtigkeitsausschluß vorgelegt und tropfenweise unter Rühren mit 30,96 g (189 mmol) 2-(4-Methylphenyl)essigsäuremethylester [Synthese: J.B. Lambert, H.W. Mark und E.S. Magyar, J. Am. Chem. Soc. 99, 3059 (1977)] und 28,10 g (189 mmol) Cyclopentylbromid in 300 ml Dimethylformamid versetzt. Man rührt 18 h bei 20°C nach und dampft das Lösemittel im Vakuum größtenteils ab. Das Rohgemisch wird mit Wasser aufgenommen und mehrfach mit Ether extrahiert. Nach Trocknung der vereinigten organischen Phasen mit Natriumsulfat engt man im Vakuum ein und erhält 41,04g (177 mmol) Produkt.
$R_f$ = 0,57 (C)

Beispiel II

2-Cyclopentyl-2-(3-methylphenyl)essigsäuremethylester

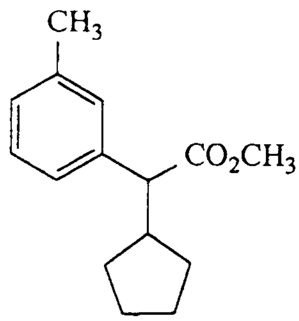

In Analogie zur Vorschrift des Beispiels I wird die Titelverbindung hergestellt.
$R_f$ = 0,56 (C)

Beispiel III

4-(1-Cyclopentyl-2-hydroxy-ethyl)-toluol

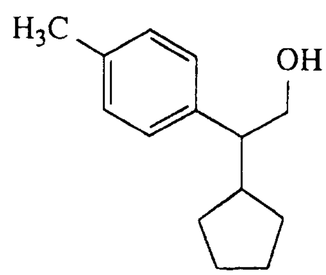

40,98 g (176 mmol) der Verbindung aus Beispiel I werden in 400 ml Tetrahydrofuran gelöst und unter Feuchtigkeitsausschluß bei 0 °C mit 3.35 g (88 mmol) Lithiumalanat versetzt. Die Reaktionsmischung wird 1,5 h bei 20 °C gerührt und bei noch vorhandenem Ausgangsmaterial (DC-Kontrolle: C) mit weiteren 3,35 g (88 mmol) Lithiumalanat umgesetzt. Nach 18 h bei 20 °C hydrolysiert man vorsichtig mit Wasser, säuert mit 2 M Schwefelsäure an, extrahiert mehrfach mit Ether und trocknet die vereinigten organischen Extrakte mit Natriumsulfat. Das Abdampfen des Lösemittels im Vakuum liefert 33,7 g (165 mmol) Produkt.
$R_f$ = 0,16 (C)

Beispiel IV

3-(1-Cyclopentyl-2-hydroxy-ethyl)toluol

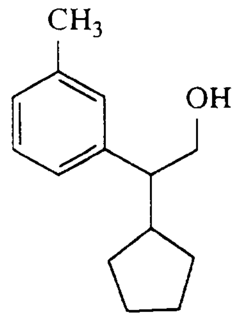

In Analogie zur Vorschrift des Beispiels III wird die Titelverbindung hergestellt.
$R_f$ = 0,19 (C)

Beispiel V

Methansulfonsäure-(2-cyclopentyl-2-(4-methylphenyl)-ethyl)ester

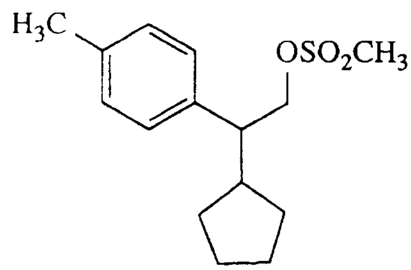

26,21 g (128 mmol) der Verbindung aus Beispiel III werden mit 12,98 g (128 mmol) Triethylamin in Dichlormethan (200 ml) gelöst und bei 0 °C mit 14,70 g (128 mmol) Methansulfonsäurechlorid umgesetzt. Nach 2,5 h bei dieser Temperatur versetzt man mit 1 M Schwefelsäure, trennt die Phasen und extrahiert die organische Lösung mit je einmal mit 1 M Schwefelsäure, Wasser, wäßriger Natriumhydrogencarbonatlösung und noch einmal mit Wasser nach. Der organische Extrakt wird mit Natriumsulfat getrocknet und liefert nach Abdampfen des Lösemittels 29.79 g (105 mmol) Produkt.
$R_f$ = 0,72 (Dichlormethan)

Beispiel VI

Methansulfonsäure-(2-cyclopentyl-2-(3-methylphenyl)ethyl)ester

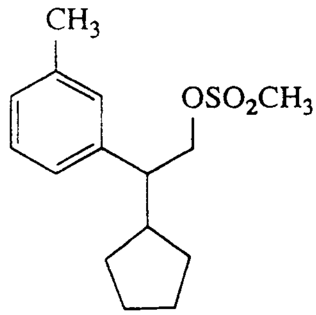

In Analogie zur Vorschrift des Beispiels V wird die Titelverbindung erhalten.
$R_f$ = 0,76 (Dichlormethan)

Beispiel VII

3-Cyclopentyl-3-(4-methylphenyl)-propionitril

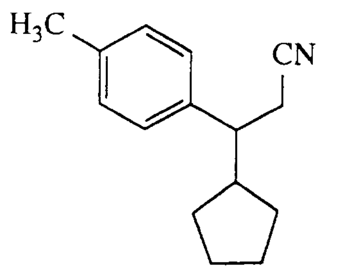

19,6 g (69 mmol) der Verbindung aus Beispiel V werden mit 3,92 g (83 mmol) Natriumcyanid in 400 ml Dimethylsulfoxid bei 90 °C zur Reaktion gebracht. Nach 45 Minuten bei dieser Temperatur verdünnt man mit Ether, wäscht mit Eisenchloridlösung und Wasser, trocknet die organische Phase mit Natriumsulfat und dampft das Lösemittel ab. Das erhaltene Rohprodukt wird an Kieselgel 60 (Merck, Petrolether: Essigester = 100:1 bis 50:1) chromatographisch gereinigt. Ausbeute: 10,4 g (49 mmol)
$R_f$ = 0,23 (Petrolether : Essigester = 20:1)

Beispiel VIII

3-Cyclopentyl-3-(3-methylphenyl)propionitril

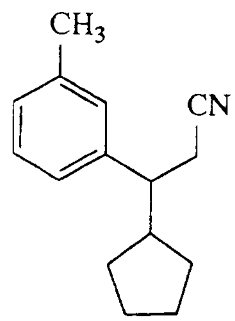

In Analogie zur Vorschrift des Beispiels VII wird die Titelverbindung hergestellt.
$R_f$ = 0,33 (C)

Beispiel IX

5-(2-Cyclopentyl)-2-(4-methylphenyl)-ethyl)tetrazol

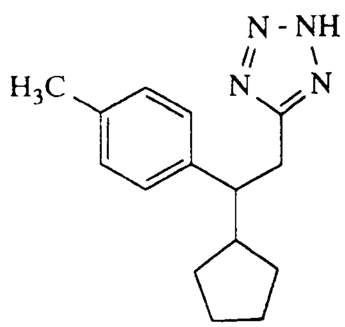

1,36 g (6,4 mmol) der Verbindung aus Beispiel VII werden in 20 ml Dimethylformamid mit 2,07 g (31,9 mmol) Natriumazid und 4,39 g (31,9 mmol) Triethylammoniumchlorid bei Rückflußtemperatur zur Reaktion gebracht Sollte nach 1 d die Reaktion unvollständig abgelaufen sein (DC-Kontrolle: F) setzt man weitere 1,04 g (15,9 mmol) Natriumazid und 2,20 g (15,9 mmol) Triethylammoniumchlorid zu und kocht weitere 24 h unter Rückfluß. Zur Aufarbeitung gießt man in 1 M Salzsäure/Ether, trocknet die organische Phase mit Natriumsulfat und dampft vom Lösemittel ab. Das erhaltene Rohprodukt wird mit Dichlormethan ausgerührt, abgesaugt und im Vakuum vom Restlösemittel befreit.
Ausbeute: 1,15g (4,5 mmol)
$R_f$ = 0,44 (F)

Beispiel X

5-(2-Cyclopentyl)-2-(3-methylphenyl)-ethyl)-tetrazol

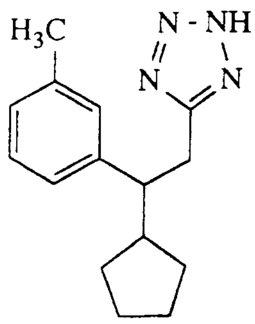

In Analogie zur Vorschrift des Beispiels IX wird die Titelverbindung erhalten.
$R_f$ = 0,40 (F)

Beispiel XI

5-(2-Cyclopentyl-2-(4-methylphenyl)-ethyl)-2-triphenylmethyl-tetrazol

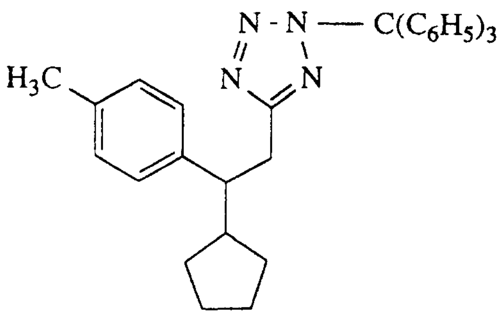

1,10 g (4.3 mmol) der Verbindung aus Beispiel IX werden in 16 ml Dichlormethan gelöst und bei 20 °C mit 1,32 g (4,7 mmol) Triphenylchlormethan und 0,56 g (5,6 mmol) Triethylamin umgesetzt. Nach 6 h wird mit 1 M wäßriger Zitronensäurelösung versetzt und mit Ether extrahiert. Die organischen Phasen werden mit Natriumsulfat getrocknet und zuletzt im Hochvakuum eingedampft
Ausbeute: 2,3 g (4,3 mmol)
$R_f$ = 0,71 (Petrolether : Essigester = 5:1)

Beispiel XII

5-(2-Cyclopentyl-2-(3-methylphenyl)-ethyl)-2-triphenylmethyl-tetrazol

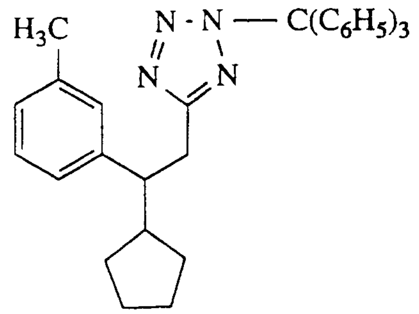

In Analogie zur Vorschrift des Beispiels XI wird die Titelverbindung hergestellt.
$R_f$ = 0,85 (Dichlormethan)

Beispiel XIII

5-(2-Cyclopentyl)-2-(4-brommethylphenyl)-ethyl)-2-triphenylmethyl-tetrazol

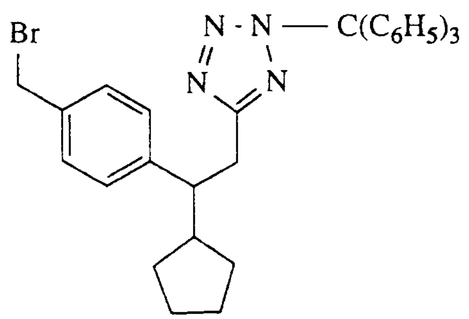

2,28 g (4.3 mmol) der Verbindung aus Beispiel XI werden in 30 ml Tetrachlormethan gelöst, mit 0,77 g (4,3 mmol) N-Bromsuccinimid und 0.1 g Azobisisobutyronitril versetzt und 6 h unter Rückfluß gekocht. Nach dem Abkühlen wird der anfallende Niederschlag abgesaugt, das Filtrat eingedampft und als Rohprodukt weiter umgesetzt.
Ausbeute: 2,6 g (4.2 mmol)
$R_f$ = 0,57 (A)
In Analogie zur Vorschrift des Beispiels XIII werden die in Tabelle I aufgeführten Verbindungen hergestellt:

Tabelle I:

| Bsp.-Nr. | | $R_f$ ( Laufmittel) |
|---|---|---|
| XIV | 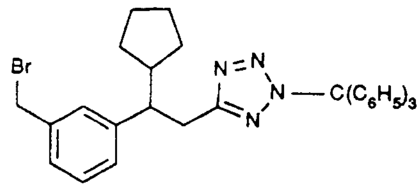 | 0,08 (I) |
| XV | 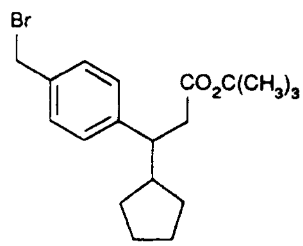 | 0,32 (C) |
| XVI | 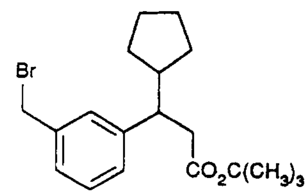 | 0,32 (K) |
| XVII | 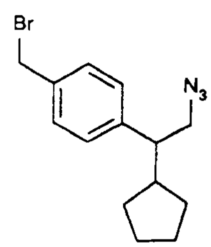 | 0,40 (K) |
| XVIII | 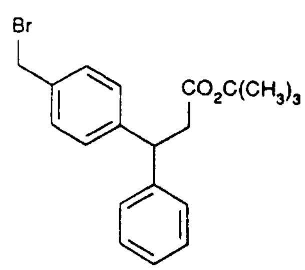 | 0,51 (C) |

Fortsetzung Tabelle I:

| Bsp.-Nr. | | $R_f$ ( Laufmittel) |
|---|---|---|
| XIX | 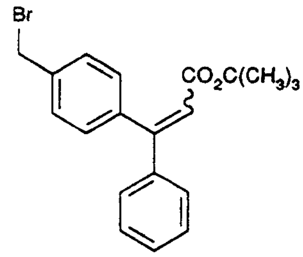 | 0,64 (C) |
| XX | 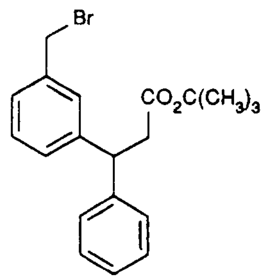 | 0,54 (C) |

Beispiel XXI

3-Cyclopentyl-3-(4-methylphenyl)propionsäure

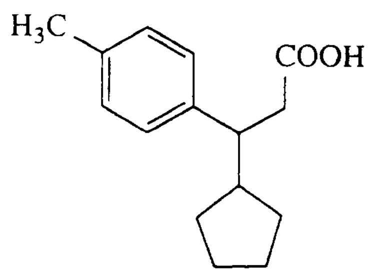

6,10 g (28,6 mmol) der Verbindung aus Beispiel VII werden in 40 ml Ethanol gelöst und mit 60 ml 1 M wäßriger Natronlauge versetzt. Das Gemisch wird 2 d unter Rückfluß gekocht, nach dem Abkühlen mit 2 M Salzsäure auf pH = 2 gestellt und mit Ether mehrfach extrahiert. Die mit Natriumsulfat getrockneten organischen Phasen werden eingedampft und das so gewonnene Rohprodukt an Kieselgel 60 (Merck, Petrolether : Essigester = 5:1, 2:1, 1:1 zuletzt 1:2) chromatographisch gereinigt. Es fallen 2,74 g (11,8 mmol) des oben genannten Produkts ($R_f$ = 0,72 E) und 2,03 g (8,8 mmol) 3-Cyclopentyl-3-(4-methylphenyl)propionsäureamid ($R_f$ = 0,14 E) an.

Beispiel XXII

3-Cyclopentyl-3-(3-methylphenyl)propionsäure

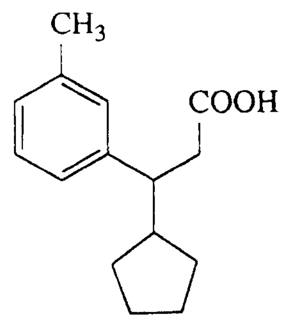

In Analogie zur Vorschrift des Beispiels XXI wird die Titelverbindung hergestellt.
$R_f$ = 0,77 (E)

Beispiel XXIII

3-Cyclopentyl-3-(4-methylphenyl)propionsäurechlorid

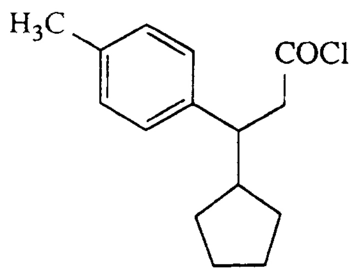

4,34 g (18,7 mmol) der Verbindung aus Beispiel XXI werden in 100 ml Toluol unter Rückfluß mit 4,98 g (39,2 mmol) Oxalylchlorid zur Reaktion gebracht.Nach 2 h wird das Lösemittel mit überschüssigem Reagenz abgedampft, das Rohprodukt wiederum in Toluol aufgenommen und erneut abgedampft. Zur völligen Entfernung des Reagenzes wird dieser Vorgang gegebenenfalls mehrfach wiederholt. Das Rohprodukt wird ohne zusätzliche Reinigung weiter umgesetzt.

Beispiel XXIV

3-Cyclopentyl-3-(3-methylphenyl)propionsäurechlorid

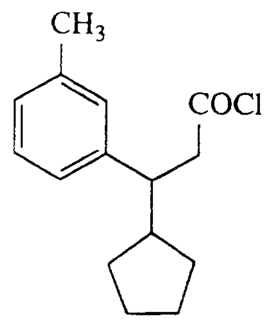

In Analogie zur Vorschrift des Beispiels XXIII wird die Titelverbindung hergestellt.

Beispiel XXV

3-Cyclopentyl-3-(4-methylphenyl)propionsäure-tert.butylester

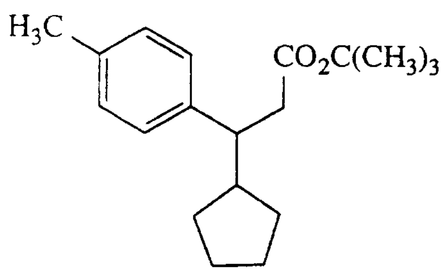

Das rohe Produkt aus Beispiel XXIII wird in 40 ml Tetrahydrofuran gelöst und bei 20 °C vorsichtig, portionsweise mit insgesamt 2.1 g (18,7 mmol) Kalium-tert.butanolat versetzt. Man rührt 2 h nach, gießt auf gesättigte, wäßrige Natriumhydrogencarbonatlösung und extrahiert mit Ether. Die gesammelten etherischen Phasen werden mit Nathumsulfat getrocknet und einrotiert. Restlösemittel wird im Hochvakuum entfernt.
Ausbeute: 5,21g (18,1 mmol)
$R_f$ = 0,80 (A)

Beispiel XXVI

3-Cyclopentyl-3-(3-methylphenyl)propionsäure-tert.butylester

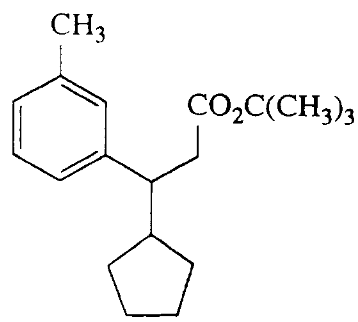

In Analogie zur Vorschrift des Beispiels XXVI wird die Titelverbindung hergestellt.
$R_f$ = 0,47 (Petrolether: Essigester = 20:1)

Beispiel XXVII

3-(3-Methylphenyl)-3-phenyl-propionsäure-tert.butylester

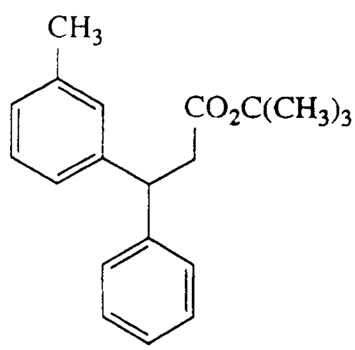

In Analogie zur Vorschrift des Beispiels XXV wird die Titelverbindung hergestellt.
$R_f$ = 0,67 (C)

Beispiel XXVIII

4-(2-Azido-1-cyclopentyl-ethyl)-toluol

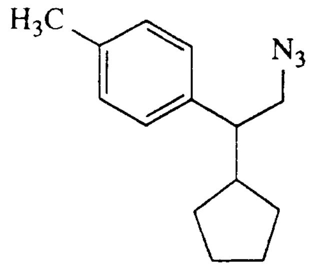

500 mg (1,8 mmol) der Verbindung aus Beispiel V werden in 10 ml Dimethylsulfoxid gelöst und bei 90 ° C mit 140 mg (2,1 mmol) Natriumazid umgesetzt. Nach 1,5 h nimmt man in 150 ml Ether und 150 ml Wasser auf und extrahiert mehrfach nach. Die organischen Phasen werden mit Natriumsulfat getrocknet und eingedampft.
Ausbeute: 370 mg (1,6 mmol)
$R_f$ = 0,69 (Petrolether : Essigester = 20:1)

Beispiel XXIX

(E,Z)-3-(4-Methylphenyl)-3-phenyl-propensäure-tert.butylester

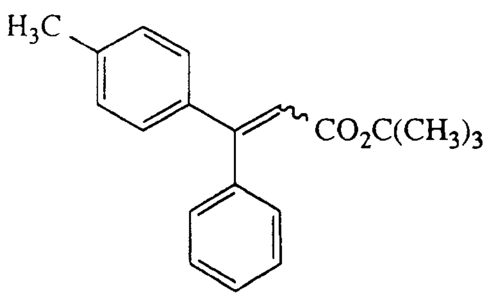

20,0 g (102 mmol) 4-Methylbenzophenon (käuflich bei Aldrich) werden in 200 ml Toluol gelöst, mit 6,95 g (231 mmol) Natriumhydrid (80%ig, stabilisiert mit Paraffin) versetzt und unter Rühren auf 80 ° C erhitzt. Eine Mischung aus 112 g (964 mmol) Essigsäure-tert.butylester (käuflich bei Aldrich) und 13,73 g (185 mmol) tert.Butanol wird bei dieser Temperatur zugetropft und das Reaktionsgemisch darauf bei 90 ° C 30 h nachgerührt. Nach dem Abkühlen werden 200 ml gesättigte wäßrige Natriumhydrogencarbonatlösung zugegeben und mehrfach mit Ether extrahiert. Die organische Phase wird mit Natriumsulfat getrocknet und eingedampft. Das Rohprodukt wird chromatographisch isoliert (Kieselgel 60, Merck, Petrolether: Dichlormethan = 10:1).
Ausbeute: 24,9 g (85 mmol)
$R_f$ = 0,67 (C)

Beispiel XXX

3-(4-Methylphenyl)-3-phenyl-propionsäure-tert.butylester

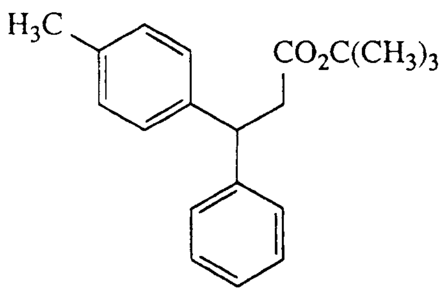

6,0 g (20,5 mmol) der Verbindung aus Beispiel XXIX werden in 200 ml Methanol gelöst und bei 20 °C mit 5,0 g (205 mmol) Magnesiumspäne zur Reaktion gebracht. Nach 3 h wird vom Niederschlag abgesaugt, das Filtrat eingedampft, mit Wasser aufgenommen und mit Ether extrahiert. Die organische Phase wird mit Natriumsulfat getrocknet und eingedampft.
Ausbeute: 5,8 g (19.6 mmol)
$R_f$ = 0,81 (C)

Beispiel XXXI

3-(3-Methylphenyl)-3-phenyl-propionsäure-N.N-diethylamid

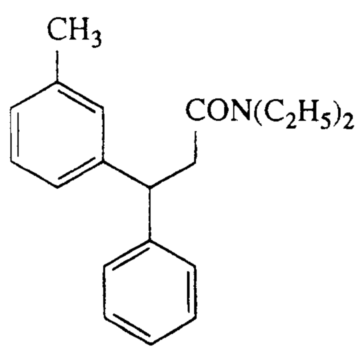

In Analogie zur Vorschrift des Beispiels XXX wird die Titelverbindung hergestellt.
$R_f$ = 0,65 (E)

Beispiel XXXII und Beispiel XXXIII

3-Hydroxy-3-(3-methylphenyl)-3-phenyl-propionsäure-N,N-diethylamid (XXXII) und (E,Z)-3-(3-Methylphenyl)-3-phenyl-propensäure-N,N-diethylamid (XXXIII)

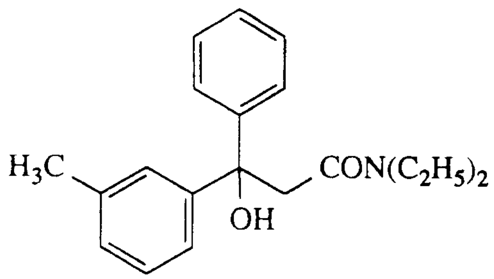

(XXXII)

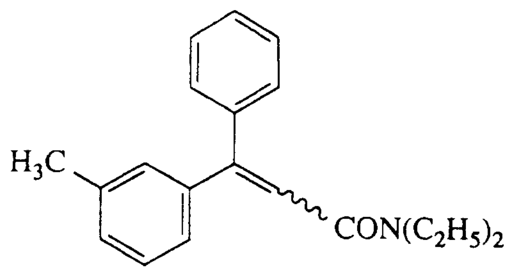

(XXXIII)

150 ml 2 M Isopropylmagnesiumchloridlösung in Ether werden bei 0 °C mit 44,8 g (612 mmol) Diethylamin versetzt und 30 Minuten unter Rückfluß gekocht, wiederum auf -5 °C gekühlt und mit einer Mischung von 20,0 g (102 mmol) 3-Methylbenzophenon (käuflich bei Aldrich) und 13,75 g (102 mmol) Essigsäure-tert.butylester (käuflich bei Aldrich) in 100 ml Ether verrührt. Man kocht 3 h unter Rückfluß und gießt nach dem Abkühlen auf Eiswasser, säuert mit Schwefelsäure an und extrahiert mehrfach mit Ether. Nach Trocknung der organischen Phase mit Natriumsulfat wird das Lösemittel abgedampft. Die chromatographische Auftrennung des Produktgemisches (Kieselgel 60, Merck, C) liefert 11,3 g (40 mmol) der Verbindung XXXII und 6,5 g (23 mmol) der Verbindung XXXIII.
$R_f$ = 0,57 (A) XXXII
$R_f$ = 0,11 (A) XXXIII

Beispiel XXXIV

3-(3-Methylphenyl)-3-phenyl-propionsäure

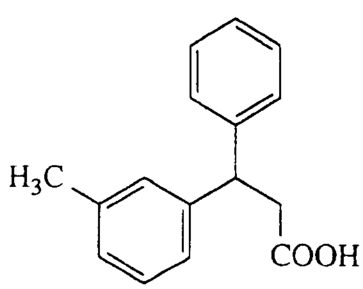

10,1 g (35,8 mmol) der Verbindung aus Beispiel XXXII werden in 200 ml Dichlormethan gelöst, vorsichtig mit 33,27 g (286,2 mmol) Triethylsilan und 130,5 g (1,14 mol) Trifluoressigsäure versetzt und 4 h

bei 20 °C gerührt. Man gibt Wasser hinzu und trennt die Phasen. Die organische Lösung wird einrotiert und der Rückstand in 1 M Natronlauge aufgenommen (pH = 12). Die alkalische wäßrige Phase wird mit Dichlormethan gewaschen, mit Salzsäure auf pH = 2 gestellt und der anfallende Niederschlag abgesaugt und mit Wasser gewaschen. Nach Trocknung im Hochvakuum über Phosphorpentoxid und Natriumhydroxid fallen 6,6 g (27,5 mmol) Produkt an.

$R_f$ = 0,28 (F)

Beispiel XXXV

N-(2-(4-Tolyl)-2-cyclopentyl-ethyl)-methansulfonsäurechlorid

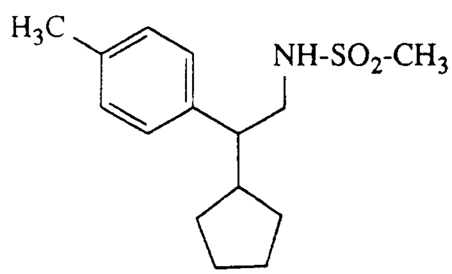

In Analogie zur Vorschrift des später aufgeführten Beispiels 45 wird die Titelverbindung erhalten.

Ausbeute: 0,065 g (0,2 mmol)

$R_f$ = 0,79 (I)

Die in Tabelle II aufgeführten Verbindungen werden in Analogie zu der Herstellungsvorschrift des Beispiels XXXV hergestellt:

Tabelle II:

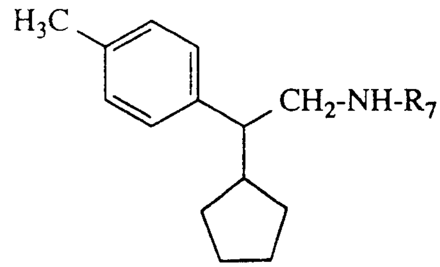

| Bsp.-Nr. | $R^7$ | $R_f$ (Laufmittel) |
|---|---|---|
| XXXVI | 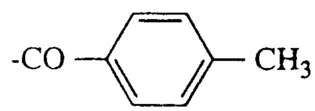 | 0,87 (I) |
| XXXVII | $-CO-CH_3$ | 0,32 (I) |
| XXXVIII | 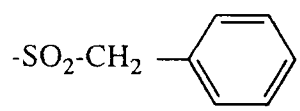 | 0,93 (I) |
| XXXIX | $-SO_2-CF_3$ | 0,93 (I) |

Herstellungsbeispiele

Beispiel 1

5-(2-[4-(2-Butyl-4-chlor-5-formyl-imidazol-1-yl-methyl)phenyl]-2-cyclopentyl-ethyl)-2-triphenylmethyl-tetrazol

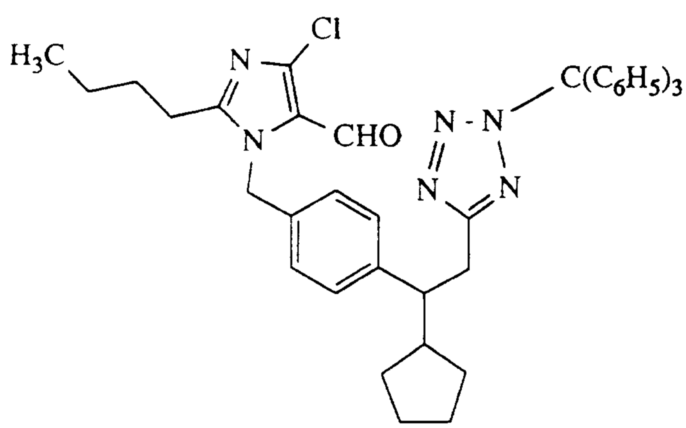

0,80 g (4,3 mmol) 2-Butyl-4-chlor-imidazol-5-carbaldehyd [Synthese: EP 324 377] werden in 10 ml Dimethylformamid gelöst und bei 0 °C mit 129 mg (4,3 mmol) Natriumhydrid (80%ig, stabilisiert mit Paraffin) deprotoniert. 15 Minuten nach beendeter Gasentwicklung werden 2,64 g (4,3 mmol) der Verbindung aus Beispiel XIII in 3 ml Dimethylformamid zugetropft, und die Reaktionsmischung wird 1 d bei 20 °C

nachgerührt. Der Zugabe von Wasser folgt die Extraktion mit Ether und die Trocknung der organischen Phase mit Natriumsulfat. Der beim Eindampfen der Etherlösung erhaltene Rückstand wird an Kieselgel 60 (Merck, Laufmittel C) chromatographisch gereinigt und liefert 1,80 g (2,5 mmol) Produkt.

$R_f$ = 0,52 (A)

In Analogie zur Vorschrift des Beispiels 1 werden die in Tabelle 1 aufgeführten Beispiele hergestellt:

Tabelle 1:

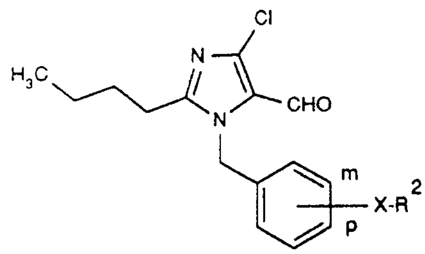

| Bsp.-Nr. | Position | X | $R^5$ | $R^2$ | $R_f$ (Laufmittel) |
|---|---|---|---|---|---|
| 2 | m | $-CHR^5-CH_2-$ | 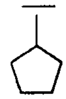 | 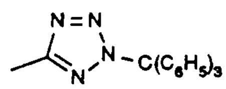 | 0,55 (A) |
| 3 | p | $-CHR^5-CH_2-$ | 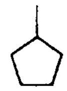 | $-CO_2C(CH_3)_3$ | 0,25 (A) |
| 4 | m | $-CHR^5-CH_2-$ | 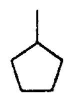 | $-CO_2C(CH_3)_3$ | 0,52 (B) |
| 5 | p | $-CHR^5-CH_2-$ | 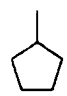 | $-N_3$ | 0,09 (C) |
| 6 | p | (E)-$CR^5$=CH- | 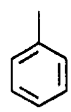 | $-CO_2C(CH_3)_3$ | 0,63 (D) |
| 7 | p | (Z)-$CR^5$=CH- | 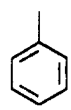 | $-CO_2C(CH_3)_3$ | 0,59 (D) |
| 8 | p | $-CHR^5-CH_2-$ | 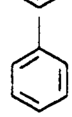 | $-CO_2C(CH_3)_3$ | 0,32 (A) |
| 9 | m | $-CHR^5-CH_2-$ | 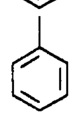 | $-CO_2C(CH_3)_3$ | 0,40 (A) |

Beispiel 10

5-(2-[4-(2-Butyl-4-chlor-5-hydroxymethyl-imidazol-1-yl-methyl)phenyl]-2-cyclopentyl-ethyl)-2-triphenylmethyl-tetrazol

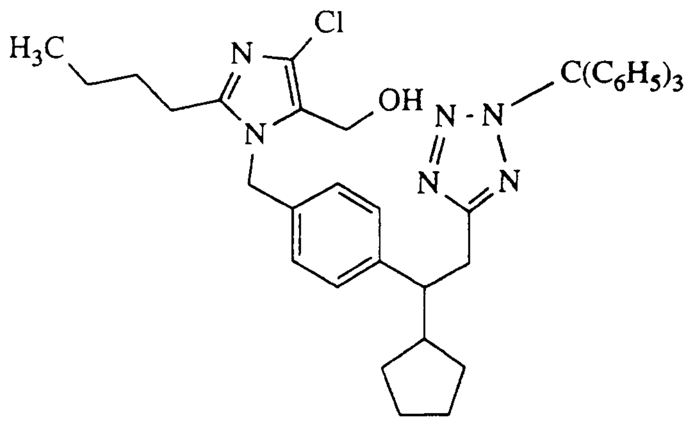

530 mg (0,74 mmol) der Verbindung aus Beispiel 1 werden bei 20 °C in 2 ml Methanol mit 27 mg (0,70 mmol) Natriumboranat umgesetzt. Nach 4 h dampft man ein und reinigt das Rohprodukt chromatographisch über Kieselgel 60 (Merck, Laufmittel D).

Ausbeute: 392 mg (0,54 mmol)

$R_f$ = 0,17 (D)

In Analogie zur Vorschrift des Beispiels 10 werden die in Tabelle 2 aufgeführten Beispiele hergestellt:

Tabelle 2:

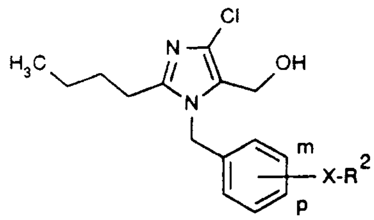

| Bsp.-Nr. | Position | X | $R^5$ | $R^2$ | $R_f$ (Laufmittel) |
|---|---|---|---|---|---|
| 11 | m | $-CHR^5-CH_2-$ | 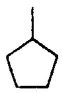 | 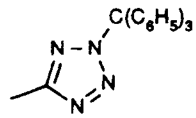 | 0,41 (E) |
| 12 | p | $-CHR^5-CH_2-$ | 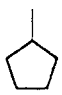 | $-CO_2C(CH_3)_3$ | 0,71 (E) |
| 13 | p | $-CHR^5-CH_2-$ | 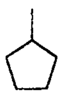 | $-N_3$ | 0,25 (F) |
| 14 | m | $CHR^5-CH_2-$ | 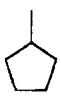 | $-CO_2C(CH_3)_3$ | 0,36 (F) |
| 15 | p | (E)-$CR^5$=CH- | 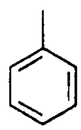 | $-CO_2C(CH_3)_3$ | 0,25 (D) |
| 16 | p | (Z)-$CR^5$=CH- | 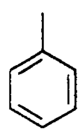 | $-CO_2C(CH_3)_3$ | 0,21 (D) |
| 17 | m | $-CHR^5-CH_2-$ | 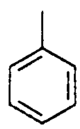 | $-CO_2C(CH_3)_3$ | 0,54 (E) |

Fortsetzung Tabelle 2:

| Bsp.-Nr. | Position | X | $R^5$ | $R^2$ | $R_f$ (Laufmittel) |
|---|---|---|---|---|---|
| 18 | p | $-CHR^5-CH_2-$ | 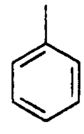 | $-CO_2C(CH_3)_3$ | 0,26 (D) |
| 19 | p | (E)-$CR^5$=CH- | 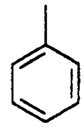 | 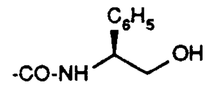 | 0,16 (F) |
| 20 | p | (Z)-$CR^5$=CH- | 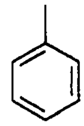 | 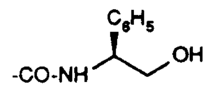 | 0,15 (F) |
| 21 | p | $HR^5-CH_2-$ | 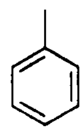 | 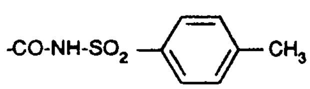 | 0,44 (F) |

Beispiel 22

5-(2-[4-(2-Butyl-4-chlor-5-formyl-imidazol-1-yl-methyl)phenyl]-2-cyclopentyl-ethyl)-tetrazol

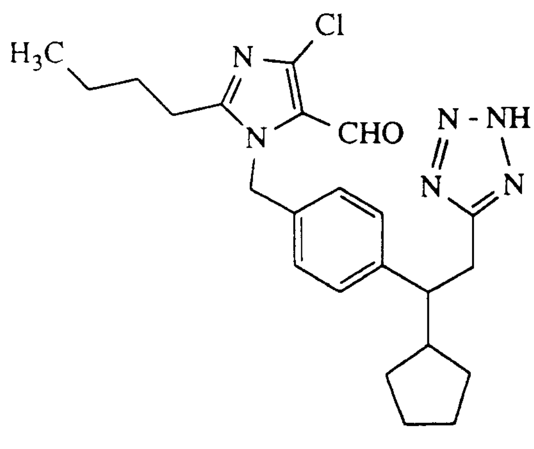

134 mg (0,19 mmol) der Verbindung aus Beispiel 1 werden in 2 ml Tetrahydrofuran mit 0,8 ml Trifluoressigsäure und 0,8 ml Wasser zur Reaktion gebracht. Nach 2 h bei 20 °C stellt man mit 2 M Natronlauge auf pH = 13, trennt die wäßrige Phase ab und zieht am Rotationsverdampfer organisches Restlösemittel aus der wäßrigen Phase ab. Bei 0 °C stellt man mit 2 M Salzsäure auf pH = 1,6, saugt den anfallenden Niederschlag ab, wäscht mit Wasser nach und trocknet das Produkt im Hochvakuum über Phosphorpentoxid und Natriumhydroxid.

Ausbeute: 51 mg (0,11 mmol)

$R_f$ = 0,47 (F)

In Analogie zur Vorschrift des Beispiels 22 werden die in Tabelle 3 aufgeführten Beispiele hergestellt:

Tabelle 3:

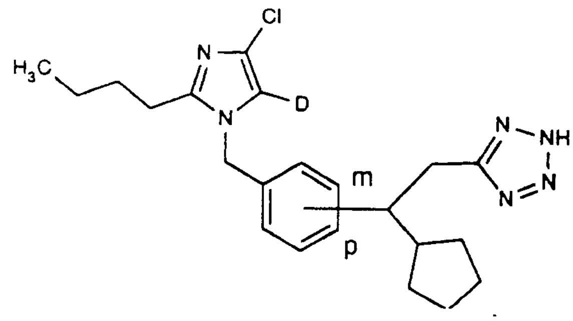

| Bsp.-Nr. | Position | D | $R_f$ (Laufmittel) |
|---|---|---|---|
| 23 | p | $-CH_2OH$ | 0,04 (F) |
| 24 | m | -CHO | 0,20 (F) |
| 25 | m | $-CH_2OH$ | 0,06 (F) |

Beispiel 26

3-[4-(2-Butyl-4-chlor-5-hydroxymethyl-imidazol-1-yl-methyl)phenyl]-3-cyclopentyl-propionsäure

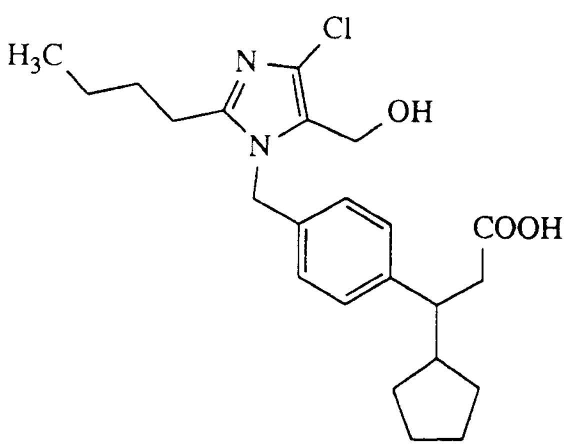

0,66 g (1,4 mmol) 3-[4-(2-Butyl-4-chlor-5-hydroxymethyl-imidazol-1-yl-methyl)phenyl]-3-cyclopentyl-propionsäure-tert.butylester werden in 20 ml 1,4-Dioxan gelöst und mit 2 ml konzentrierter Salzsäure versetzt. Das Gemisch wird 20 h bei 20 °C gerührt, mit Ether und 2 M Salzsäure verdünnt und die Phasen getrennt Die organische Phase wird eingedampft, in wäßriger Natronlauge (pH = 11) aufgenommen und mit Ether gewaschen. Darauf wird das organische Restlösemittel der wäßrigen Phase am Rotationsverdampfer im Vakuum entfernt und das Produkt bei 0 °C mit 2 M Salzsäure bei pH = 2 gefällt, abgesaugt, mit Wasser gewaschen und im Hochvakuum über Natriumhydroxid und Phosphorpentoxid getrocknet.

Ausbeute: 0,46 g (1,1 mmol)

$R_f$ = 0,16 (F)

In Analogie zur Vorschrift des Beispiels 26 werden die in Tabelle 4 aufgeführten Beispiele hergestellt:

Tabelle 4:

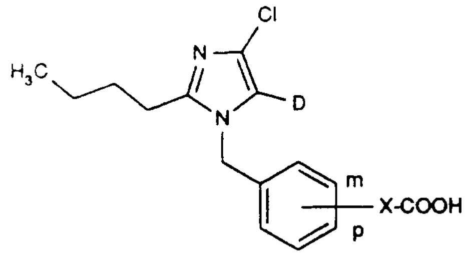

| Bsp.-Nr. | Position | X | $R^5$ | D | $R_f$ (Laufmittel) |
|---|---|---|---|---|---|
| 27 | m | $-CHR^5-CH_2-$ | 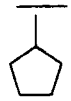 | -CHO | 0,29 (F) |
| 28 | m | $-CHR^5-CH_2-$ | 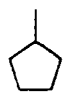 | $-CH_2OH$ | 0,17 (G) |
| 29 | p | (E)-$CR^5$=CH- | 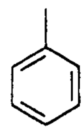 | $-CH_2OH$ | 0,56 (H) |
| 30 | p | (Z)-$CR^5$=CH- | 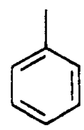 | $-CH_2OH$ | 0,56 (H) |
| 31 | p | (E)-$CR^5$=CH- | 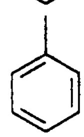 | -CHO | 0,59 (G) |
| 32 | m | $-CHR^5-CH_2-$ | 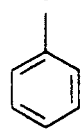 | -CHO | 0,28 (G) |
| 33 | m | $-CHR^5-CH_2-$ | 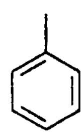 | $-CH_2OH$ | 0,18 (G) |

Fortsetzung Tabelle 4:

| Bsp.-Nr. | Position | X | $R^5$ | D | $R_f$ (Laufmittel) |
|---|---|---|---|---|---|
| 34 | p | (Z)-$CR^5$=CH- | 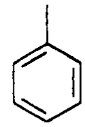 | CHO | 0,54 (G) |
| 35 | p | -$CHR^5$-$CH_2$- | 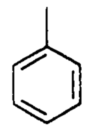 | CHO | 0,19 (F) |
| 36 | p | -$CHR^5$-$CH_2$- | 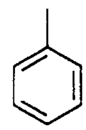 | $CH_2OH$ | 0,43 (G) |
| 37 | p | -$CHR^5$-$CH_2$- | 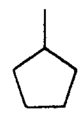 | CHO | 0,40 (F) |

Beispiel 38

3-[4-(2-Butyl-4-chlor-5-formyl-imidazol-1-yl-methyl)phenyl]-3-phenyl-propionsäure-N-(4-tolylsulfonyl)amid

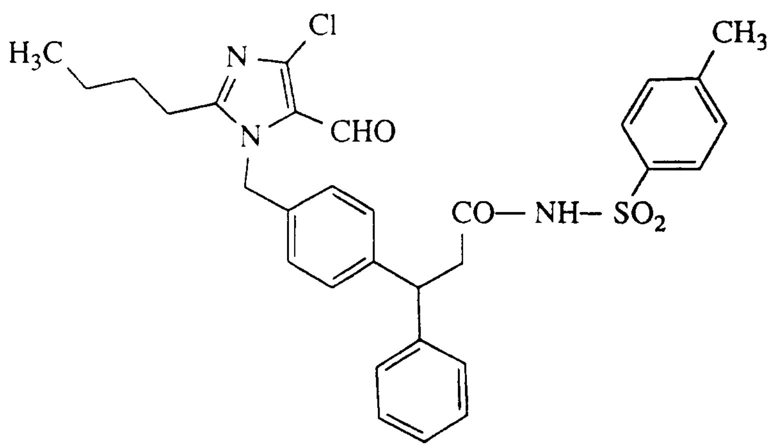

0,30 g (0,7 mmol) 3-[4-(2-Butyl-4-chlor-5-formyl-imidazol-1-yl-methyl)phenyl]-3-phenyl-propionsäure werden in 15 ml Tetrahydrofuran bei -20 °C mit 60 µl (0,77 mmol) Methansulfonsäurechlorid und 428 µl (3,10 mmol) Triethylamin umgesetzt. Nachdem das Gemisch 2 h bei -20 °C gerührt worden ist, werden 148 mg (0,85 mmol) 4-Toluolsulfonsäureamid und 343 mg (2,82 mmol) 4-(N,N-Dimethylamino)pyridin zugegeben. Die Reaktionstemperatur steigt nach Entfernen des Kühlbades auf 20 °C, und man rührt 1 d nach. Darauf wird wäßrige Natriumhydrogencarbonatlösung zugestzt und mit Ether exkahiert. Die organische Lösung wird mit Natriumsulfat getrocknet, eingedampft und der Rückstand a Kieselgel 60 (Merck, Dichlormethan : Methanol = 100:1) chromatographisch gereinigt.

Ausbeute: 0,24 g (0,4 mmol)

$R_f$ = 0,62 (F)

In Analogie zur Vorschrift des Beispiels 38 werden die in Tabelle 5 aufgeführten Beispiele hergestellt:

Tabelle 5:

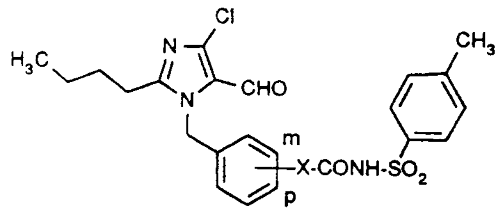

| Bsp.-Nr. | Position | X | $R^5$ | $R_f$ (Laufmittel) |
|---|---|---|---|---|
| 39 | p | (E)-$CR^5$=CH- | 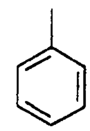 | 0,66 (E) |
| 40 | p | (Z)-$CR^5$=CH- | 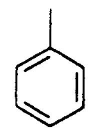 | 0,56 (E) |

Beispiel 41

3-[4-(2-Butyl-4-chlor-5-formyl-imidazol-1-yl-methyl)phenyl]-3-phenylpropionsäure-L-phenylglycinol-amid

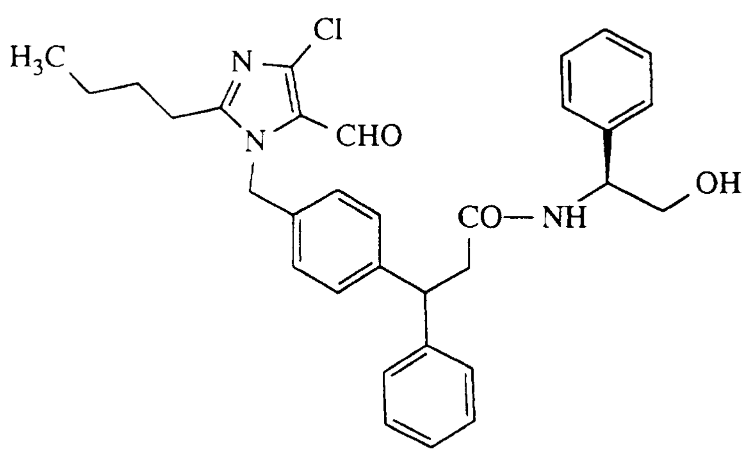

0,30 g (0,7 mmol) 3-[4-(2-Butyl-4-chlor-5-formyl-imidazol-1-yl-methyl)phenyl]-3-phenyl-propionsäure werden in 15 ml Tetrahydrofuran bei -20°C mit 60 µl (0,77 mmol) Methansulfonylchlorid und 428 µl (3,10 mmol) Triethylamin umgesetzt. Nach einer Rührzeit von 2 h bei -20°C setzt man 0,12 g (0,84 mmol) L-Phenylglycinol und 34,3 mg (2,82 mmol) 4-(N,N-Dimethylamino)pyridin zu und rührt unter Erwärmung auf 20°C 20 h nach. Die Mischung wird mit wäßriger Natriumhydrogencarbonatlösung auf pH = 9 gestellt und mit Ether extrahiert. Die organische Phase wird mit Natriumsulfat getrocknet, eingedampft und der Rückstand an Kieselgel 60 (Merck, Dichlormethan : Methanol = 100:1) chromatographisch gereinigt.
Ausbeute: 0,12 g (0,2 mmol)
$R_f$ = 0,62 (F)
In Analogie zur Vorschrift des Beispiels 41 werden die in Tabelle 6 aufgeführten Verbindungen hergestellt:

Tabelle 6:

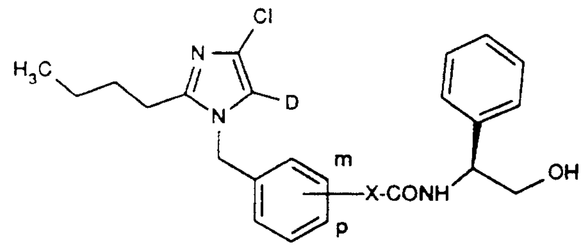

| Bsp.-Nr. | Position | X | $R^5$ | D | $R_f$ (Laufmittel) |
|---|---|---|---|---|---|
| 42 | p | (E)-$CR^5$=CH- | 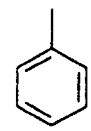 | CHO | 0,79 (G) |
| 43 | p | (Z)-$CR^5$=CH- | 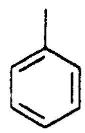 | CHO | 0,68 (G) |

Beispiel 44

1-(4-(2-Amino-1-cyclopentyl-ethyl)phenyl-methyl)-2-butyl-5-hydroxymethyl-imidazol

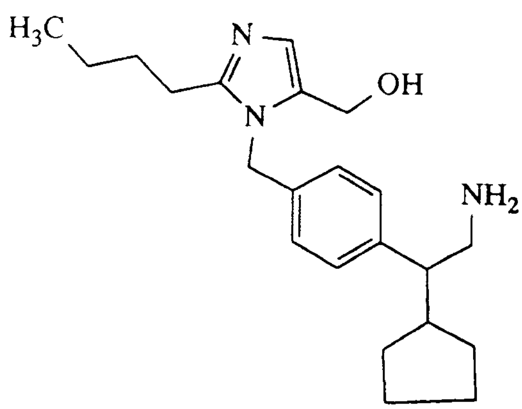

1,21 g (2.9 mmol) 1-(4-(2-Azido-1-cyclopentylethyl)phenyl-methyl)-2-butyl-4-chlor-5-hydroxymethyl-imidazol werden bei 20 °C in 15 ml Methanol mit 1.2 g Palladium (10% auf Tierkohle) und Wasserstoffatmosphäre gerührt. Nach 3 h wird der Katalysator abfiltriert und das Filtrat - zuletzt im Hochvakuum - eingedampft.

Ausbeute: 1.07 g (2.7 mmol) Produkt, das roh weiterverarbeitet wird

$R_f$ = 0,57 (Dichlormethan : Methanol = 2:1)

Beispiel 45

1-[4-(2-Cyclopentyl-1-(N-methylsulfonyl-amino)ethyl)phenyl-methyl]-2-butyl-5-hydroxymethyl-imidazol

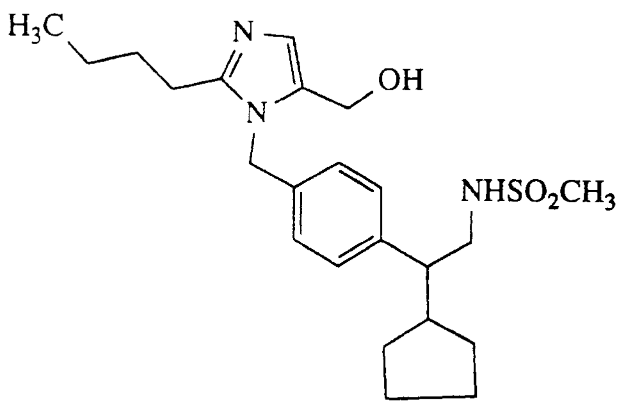

390 mg (1,0 mmol) der Verbindung aus Beispiel 44 werden in 4 ml Dichlormethan gelöst und unter Eiskühlung mit 0,14 ml (1,0 mmol) Triethylamin und 0,08 ml (1,00 mmol) Methansulfonsäurechlorid umgesetzt. Nach beendeter Zugabe wird 4 h nachgerührt und dann mit 1 M Schwefelsäure und Ether extrahiert. Die organische Phase wird mit Natriumsulfat getrocknet, eingedampft und der Rückstand an Kieselgel 60 (Merck, Dichlormethan : Methanol 50:1, 20:1, 1:1 zu Methanol) chromatographisch gereinigt.
Ausbeute: 68 mg (0.2 mmol)
$R_f$ = 0,03 (I)
In Analogie zur Vorschrift des Beispiels 45 werden die in Tabelle 7 aufgeführten Beispiele hergestellt:

Tabelle 7:

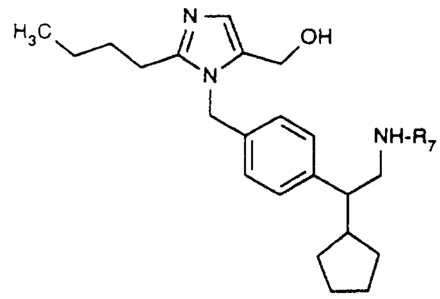

| Bsp.-Nr. | $R^7$ | $R_f$ (Laufmittel) |
|---|---|---|
| 46 | 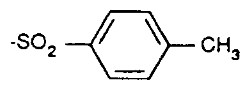 | 0,81 (I) |
| 47 | 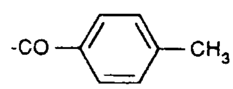 | 0,43 (I) |
| 48 | $-CO-CH_3$ | 0,01 (I) |
| 49 | 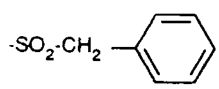 | 0,85 (I) |
| 50 | $-SO_2-CF_3$ | 0,86 (I) |

**Patentansprüche**

1. Imidazolyl-substituierte Phenylpropion- und -zimtsäurederivate der allgemeinen Formel

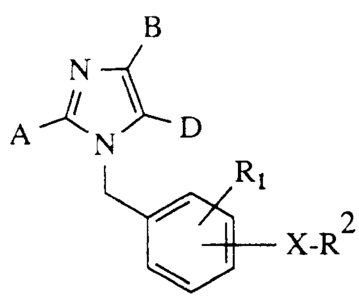

(I),

in welcher

| | |
|---|---|
| A | für geradkettiges oder verzweigtes Alkyl oder Alkenyl mit jeweils bis zu 8 Kohlenstoffatomen steht, oder für Cycloalkyl mit 3 bis 8 Kohlenstoffatomen steht, |
| B | für Wasserstoff, Halogen oder für Perfluoralkyl mit bis zu 5 Kohlenstoffatomen steht, |

D für eine Gruppe der Formel $-CH_2-OR^3$ oder $-CO-R^4$ steht,

worin

$R^3$ Wasserstoff oder geradkettiges oder verzweigtes Alkyl mit bis zu 8 Kohlenstoffatomen bedeutet,

$R^4$ Wasserstoff, Hydroxy oder geradkettiges oder verzweigtes Alkoxy mit bis zu 8 Kohlenstoffatomen bedeutet,

X für eine Gruppe der Formel

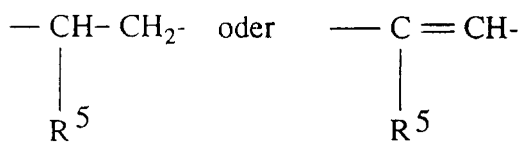

steht,

worin

$R^5$ Cycloalkyl mit 3 bis 8 Kohlenstoffatomen bedeutet, oder Phenyl bedeutet,

oder

geradkettiges oder verzweigtes Alkyl mit bis zu 8 Kohlenstoffatomen bedeutet, das gegebenenfalls durch Phenyl oder Cycloalkyl mit 3 bis 8 Kohlenstoffatomen substituiert ist,

$R^1$ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

$R^2$ für Carboxy, geradkettiges oder verzweigtes Alkoxycarbonyl mit bis zu 8 Kohlenstoffatomen oder Azido steht, oder

für einen Rest der Formel $-NR^6R^7$, $-CO-NR^8R^9$ oder

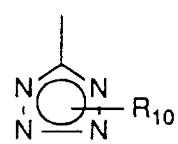

steht,

worin

$R^6$, $R^7$, $R^8$ und $R^9$ gleich oder verschieden sind und Wasserstoff, geradkettiges oder verzweigtes Alkyl mit bis zu 8 Kohlenstoffatomen, Benzyl oder Phenyl bedeuten,

oder

$R^6$ und $R^8$ die oben angegebene Bedeutung haben

und

$R^7$ und/oder $R^9$ eine Gruppe der Formel $-SO_2R^{11}$ oder $-CO-R^{12}$ bedeuten,

worin

$R^{11}$ Trifluormethyl oder geradkettiges oder verzweigtes Alkyl mit bis zu 8 Kohlenstoffatomen bedeutet, das gegebenenfalls durch Phenyl substituiert ist, das seinerseits durch geradkettiges oder verzweigtes Alkyl mit bis zu 6 Kohlenstoffatomen substituiert sein kann, oder

Phenyl bedeutet, das gegebenenfalls durch geradkettiges oder verzweigtes Alkyl mit bis zu 6 Kohlenstoffatomen substituiert ist,

$R^{12}$ geradkettiges oder verzweigtes Alkyl mit bis zu 8 Kohlenstoffatomen, Benzyl oder Phenyl bedeutet, das gegebenenfalls durch geradkettiges oder verzweigtes Alkyl mit bis zu 6 Kohlenstoffatomen substituiert ist,

oder

$R^8$ Wasserstoff bedeutet

und

$R^9$ die Gruppe der Formel

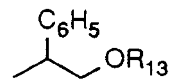

bedeutet,
worin

$R^{13}$ Wasserstoff oder geradkettiges oder verzweigtes Alkyl mit bis zu 6 Kohlenstoffatomen bedeutet,

$R^{10}$ Wasserstoff, geradkettiges oder verzweigtes Alkyl mit bis zu 6 Kohlenstoffatomen oder die Triphenylmethylgruppe bedeutet,

und deren Salze.

**2.** Imidazolyl-substituierte Phenylpropion- und -zimtsäurederivate nach Anspruch 1,
wobei

A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,

B für Wasserstoff, Fluor, Chlor, Brom oder für Perfluoralkyl mit bis zu 4 Kohlenstoffatomen steht,

D für eine Gruppe der Formel $-CH_2OR^3$ oder $-CO-R^4$ steht,
worin

$R^3$ Wasserstoff oder geradkettiges oder verzweigtes Alkyl mit bis zu 6 Kohlenstoffatomen bedeutet,

$R^4$ Wasserstoff, Hydroxy oder geradkettiges oder verzweigtes Alkoxy mit bis zu 6 Kohlenstoffatomen bedeutet,

X für eine Gruppe der Formel

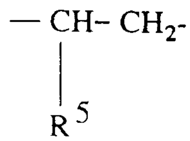 oder 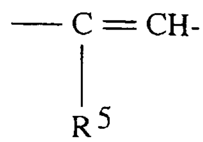

steht,
worin

$R^5$ Cyclopropyl, Cyclopentyl, Cyclohexyl, Cycloheptyl oder Phenyl bedeutet,
oder
geradkettiges oder verzweigtes Alkyl mit bis zu 6 Kohlenstoffatomen bedeutet, das durch Phenyl, Cyclopropyl, Cyclopentyl, Cyclohexyl oder Cycloheptyl substituiert ist,

$R^1$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

$R^2$ für Carboxy, geradkettiges oder verzweigtes Alkoxycarbonyl mit bis zu 6 Kohlenstoffatomen oder Azido steht, oder
für einen Rest der Formel $-NR^6R^7$, $-CO-NR^8R^9$ oder

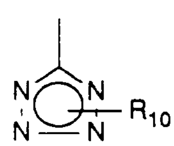

steht,
worin

$R^6$, $R^7$, $R^8$ und $R^9$ gleich oder verschieden sind und Wasserstoff, geradkettiges oder verzweigtes Alkyl mit bis zu 6 Kohlenstoffatomen, Benzyl oder Phenyl bedeuten,

| | |
|---|---|
| | oder |
| $R^6$ und $R^8$ | die oben angegebene Bedeutung haben |
| | und |
| $R^7$ und oder $R^8$ | eine Gruppe der Formel $-SO_2R^{11}$ oder $-CO-R^{12}$ bedeuten, |
| | worin |
| $R^{11}$ | Trifluormethyl oder geradkettiges oder verzweigtes Alkyl mit bis zu 6 Kohlenstoffatomen bedeutet, das gegebenenfalls durch Phenyl substituiert ist, das seinerseits durch geradkettiges oder verzweigtes Alkyl mit bis zu 4 Kohlenstoffatomen substituiert sein kann, oder |
| | Phenyl bedeutet, das gegebenenfalls durch geradkettiges oder verzweigtes Alkyl mit bis zu 4 Kohlenstoffatomen substituiert ist, |
| $R^{12}$ | geradkettiges oder verzweigtes Alkyl mit bis zu 6 Kohlenstoffatomen, Phenyl, Benzyl oder Tolyl bedeutet, |
| | oder |
| $R^8$ | Wasserstoff bedeutet |
| | und |
| $R^9$ | die Gruppe der Formel |

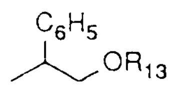

| | |
|---|---|
| | bedeutet, |
| | worin |
| $R^{13}$ | Wasserstoff oder geradkettiges oder verzweigtes Alkyl mit bis zu 4 Kohlenstoffatomen bedeutet, |
| $R^{10}$ | Wasserstoff geradkettiges oder verzweigtes Alkyl mit bis zu 4 Kohlenstoffatomen oder die Triphenylmethylgruppe bedeutet |

und deren Salze.

**3.** Imidazolyl-substituierte Phenylpropion- und -zimtsäurederivate nach Anspruch 1, wobei

| | |
|---|---|
| A | für geradkettiges oder verzweigtes Alkyl oder Alkenyl mit jeweils bis zu 4 Kohlenstoffatomen steht, oder |
| | für Cyclopropyl, Cyclopentyl oder Cyclohexyl steht, |
| B | für Wasserstoff, Fluor, Chlor oder für Perfluoralkyl mit bis zu 2 Kohlenstoffatomen steht, |
| D | für eine Gruppe der Formel $-CH_2OR^3$ oder $-CO-R^4$ steht, |
| | worin |
| $R^3$ | Wasserstoff oder geradkettiges oder verzweigtes Alkyl mit bis zu 4 Kohlenstoffatomen bedeutet, |
| $R^4$ | Wasserstoff, Hydroxy oder geradkettiges oder verzweigtes Alkoxy mit bis zu 4 Kohlenstoffatomen bedeutet, |
| X | für eine Gruppe der Formel |

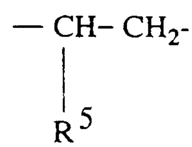
oder
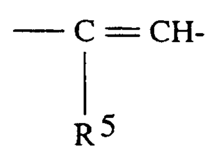

| | |
|---|---|
| | steht, |
| | worin |
| $R^5$ | Cyclopentyl, Cyclohexyl oder Phenyl bedeutet, oder |
| | geradkettiges oder verzweigtes Alkyl mit bis zu 4 Kohlenstoffatomen bedeutet, |

das gegebenenfalls durch Phenyl, Cyclopentyl oder Cyclohexyl substituiert ist,

$R^1$ für Wasserstoff, Fluor, Chlor, Brom, Trifluormethyl, Trifluormethoxy oder Methyl steht,

$R^2$ für Carboxy, geradkettiges oder verzweigtes Alkoxycarbonyl mit bis zu 4 Kohlenstoffatomen oder Azido steht, oder
für einen Rest der Formel $-NR^6R^7$, $-CO-NR^8R^9$ oder

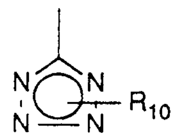

steht,
worin

$R^6$, $R^7$, $R^8$ und $R^9$ gleich oder verschieden sind und Wasserstoff, geradkettiges oder verzweigtes Alkyl mit bis zu 4 Kohlenstoffatomen, Benzyl oder Phenyl bedeuten,
oder

$R^6$ und $R^8$ die oben angegebene Bedeutung haben
und

$R^7$ und/oder $R^9$ eine Gruppe der Formel $-SO_2-R^{11}$ oder $-CO-R^{12}$ bedeuten,
worin

$R^{11}$ Trifluormethyl oder geradkettiges oder verzweigtes Alkyl mit bis zu 4 Kohlenstoffatomen bedeutet, das gegebenenfalls durch Phenyl oder Tolyl substituiert sein kann, oder
Phenyl oder Tolyl bedeutet,

$R^{12}$ geradkettiges oder verzweigtes Alkyl mit bis zu 4 Kohlenstoffatomen, Benzyl, Phenyl oder Tolyl bedeutet,
oder

$R^8$ Wasserstoff bedeutet
und

$R^9$ die Gruppe der Formel

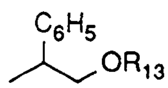

bedeutet,
worin

$R^{13}$ Wasserstoff, Methyl oder Ethyl bedeutet,

$R^{10}$ Wasserstoff, Methyl oder die Triphenylmethylgruppe bedeutet,

und deren Salze.

**4.** Imidazolyl-substituierte Phenylpropion- und -zimtsäurederivate nach Anspruch 1 zur therapeutischen Behandlung.

**5.** Verfahren zur Herstellung von Imidazolyl-substituierten Phenylpropion- und -zimtsäurederivaten nach Anspruch 1, dadurch gekennzeichnet, daß man Verbindungen der allgemeinen Formel

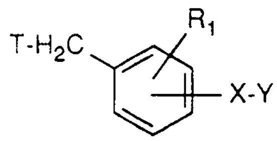

(II),

in welcher

$R^1$ und X die oben angegebene Bedeutung haben,

T für eine typische Abgangsgruppe wie beispielsweise Chlor, Brom, Jod, Tosylat oder Mesylat, vorzugsweise für Brom, steht

und

Y für geradkettiges oder verzweigtes ($C_1$-$C_4$)-Alkoxycarbonyl oder für die Triphenylmethyl-tetrazol-1-yl-Gruppe steht,

zunächst mit Imidazolen der allgemeinen Formel (III)

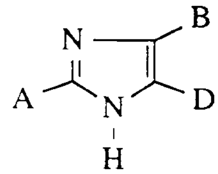

(III),

in welcher

A, B und D die oben angegebene Bedeutung haben,

in inerten Lösemitteln, gegebenenfalls in Anwesenheit einer Base und gegebenenfalls unter Schutzgasatmosphäre zu Verbindungen der allgemeinen Formel (IV)

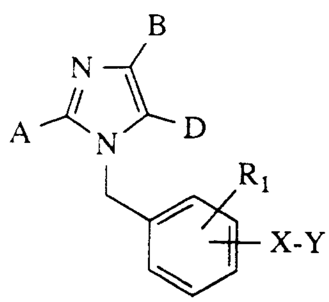

(IV),

in welcher

A, B, D, E, $R^1$ und X und Y die oben angegebene Bedeutung haben,

umsetzt,

und im Fall der Säuren ($R^2$ = $CO_2H$) die Ester verseift,

und im Fall der Amine, Amide und Sulfonamide mit Verbindungen der allgemeinen Formel (V)

$H\text{-}NR^{14}R^{15}$ (V),

in welcher

$R^{14}$ und $R^{15}$ den jeweiligen Bedeutungsumfang von $R^6$, $R^7$, $R^8$ und $R^9$ erfassen,

gegebenenfalls in Anwesenheit einer Base und/oder eines Hilfsstoffes, beispielsweise eines Dehydratisierungsmittels, in inerten Losemitteln umsetzt,

und im Fall des freien Tetrazols, die Tritylgruppe mit Säuren, vorzugsweise mit Trifluoressigsäure oder Salzsäure in Dioxan, abspaltet,

und gegebenenfalls die Substituenten A, B, D und $R^1$ nach üblichen Methoden, beispielsweise durch Reduktion, Oxidation, Alkylierung oder Hydrolyse einführt oder in andere Gruppen überführt,

und gegebenenfalls die Isomeren trennt, und im Fall der Herstellung der Salze mit einer entsprechenden Base oder Säure umsetzt. und im Fall der Ester. ausgehend von den aktivierten Carbonsäuren, mit den entsprechenden Alkoholaten umsetzt.

6. Verfahren nach Anspruch 5, dadurch gekennzeichnet, daß die Umsetzung in einem Temperaturbereich von -30 °C bis +100 °C durchgeführt wird.

7. Arzneimittel enthaltend mindestens ein Imidazolyl-substituiertes Phenylpropion- oder -zimtsäurederivat nach Anspruch 1.

8. Verwendung der Imidazolyl-substituierten Phenylpropion- und -zimtsäurederivate nach Anspruch 1 zur Herstellung von Arzneimitteln.

9. Verbindungen der allgemeinen Formel

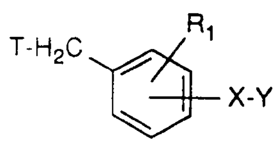

(II),

in welcher

R' und X die oben angegebene Bedeutung haben,

T für eine typische Abgangsgruppe wie beispielsweise Chlor, Brom, Jod, Tosylat oder Mesylat steht

und

Y für geradkettiges oder verzweigtes ($C_1$-$C_4$)-Alkoxycarbonyl steht.

10. Verfahren zur Herstellung von Verbindungen nach Anspruch 9, dadurch gekennzeichnet, daß man zunächst Verbindungen der allgemeinen Formel (VI)

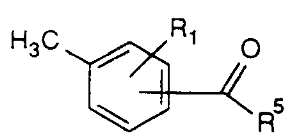

(VI),

in welcher

$R^1$ und $R^5$ die oben angegebene Bedeutung haben,

durch Reduktion nach üblichen Methoden, vorzugsweise mit Natriumhydrid, in einem der oben aufgeführten Lösemitteln, vorzugsweise in Toluol, und durch anschließende Veresterung in die Verbindungen der allgemeinen Formel (VII)

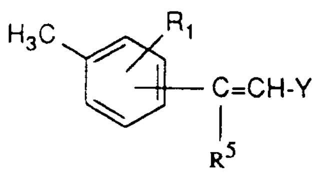

(VII),

in welcher

R', $R^5$ und Y die oben angegebene Bedeutung haben,

überführt.

und in einem zweiten Schritt eine Bromierung der Methylgruppe, gegebenenfalls in Anwesenheit eines Katalysators durchführt,
und im Fall, daß der Rest X für die

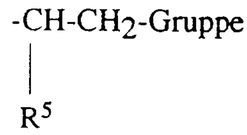

steht,
entweder Verbindungen der allgemeinen Formel (VII) nach üblichen Methoden reduziert oder zunächst Verbindungen der allgemeinen Formel (VI) in einer Grignardreaktion, beispielsweise mit ($C_1$-$C_4$)-Alkylmagnesiumhalogeniden, vorzugsweise Bromiden reduziert und gleichzeitig den Rest $R^2$ einführt und anschließend wie oben beschrieben die Bromierung durchführt,
und gegebenenfalls auf den Stufen der allgemeinen Formel (VI) oder (VII) den Substituenten $R^1$ wie oben beschrieben variiert.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 93 10 9464

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| Y | EP-A-0 169 408 (HOECHST AG) 29. Januar 1986 * Seite 1, Zeile 21 - Seite 2, Zeile 5; Ansprüche 1-5 * | 1-10 | C07D233/68 C07D403/10 A61K31/415 C07D233/54 |
| Y | DE-A-3 130 254 (A. NATTERMANN & CIE. GMBH) 17. Februar 1983 * Seite 2, Zeile 1 - Seite 2, Zeile 13; Ansprüche 1-17 * | 1-10 | |
| Y | DE-A-2 923 815 (ONO PHARMACEUTICAL CO., LTD.) 10. Januar 1980 * Seite 2, Zeile 32 - Seite 3, Zeile 3; Ansprüche 1-28 * | 1-10 | |

RECHERCHIERTE SACHGEBIETE (Int. Cl. 5)

C07D
A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| MUENCHEN | 27 OKTOBER 1993 | HERZ C.P. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

EP 93 109 464.3

- C -

Die Definition des Substituenten T in Formel II von Anspruch 9 ist zu allgemein und/oder umfaßt einen viel zu breiten Bereich chemisch verschiedener Gruppen, welche im Beschreibungsteil der Anmeldung nicht oder nur zum Teil durch Beispiele veranschaulicht werden.

Die aus diesen Definitionen ableitbare große Zahl theoretisch vorstellbarer Verbindungen, welche sich aus den beanspruchten Kombinationsmöglichkeiten ergibt, schließt eine sinnvolle, vollständige und ökonomische Recherche aus. Auch im Hinblick auf das in der Beschreibung offenbarte, der Anmeldung zugrundeliegende erfinderische Konzept wurde die Recherche auf die konkret offenbarten Ausführungsbeispiele beschränkt.

( s.a. Artikel 83, 84 EPÜ, Regel 45 I.R., Richtlinien für die Prüfung im EPA, Teil B, III-2.1, 3.6, 3.7).